# EUROPEAN PATENT SPECIFICATION

(11) **EP 4 149 589 B1**
(45) Date of publication and mention of the grant of the patent: **18.03.2026**
(21) Application number: 21734032.2
(22) Date of filing: 11.05.2021
(51) Int. Cl.: A61M 5/19, A61F 9/00

(54) **DELIVERY OF FLUID TO OPHTHALMIC PASSAGES**
ABGABE VON FLÜSSIGKEIT AN OPHTHALMISCHE DURCHGÄNGE
ADMINISTRATION DE FLUIDE À DES PASSAGES OPHTALMIQUES

(30) Priority: 11.05.2020 US 202063023162 P
(43) Date of publication of application: 22.03.2023
(73) Proprietor: Glaukos Corporation, Aliso Viejo, CA 92656 (US)
(72) Inventor: JOHNSON, Andrew David, Aliso Viejo, CA 92656 (US); HAFFNER, David Steven, Aliso Viejo, CA 92656 (US); RANGEL-FRIEDMAN, Gary, Aliso Viejo, CA 92656 (US); GUNN, Nicholas Max, Aliso Viejo, CA 92656 (US)
(74) Representative: Hoffmann Eitle
(86) International application number: PCT/US2021/031823
(87) International publication number: WO 2021/231461

(56) References cited:
- WO-A1-2016/023116
- WO-A1-2019/200336
- US-A1- 2016 287 438

## Description

### INCORPORATION BY REFERENCE TO ANY PRIORITY APPLICATIONS

This application claims priority to US Provisional Application No. 63/023,162 filed May 11, 2020.

### BACKGROUND

Delivery of small amounts of viscoelastic to Schlemm's canal, collector channels, and the downstream episcleral venous networks has been shown to lower intraocular pressure in patients with open-angle glaucoma.

Prior art useful for understanding the present invention can be found in the following documents:
US 2016 / 287 438 A1 relates to systems and methods for accessing Schlemm's canal and for delivering an ocular device, tool, or fluid composition therein.
US 2019 / 200 336 A1 relates to devices and methods for intraocular fluid injection.
WO 2016 / 023 116 A1 relates to devices and methods for the ab interno delivery of two or more fluid compositions into Schlemm's canal through a single insertion point in the eye with minimal trauma.

### SUMMARY

The present invention is defined by the appended independent claim. The dependent claims are directed to optional features and preferred embodiments.
In aspects of the present invention, there is provided a device for delivering fluid according to the independent claim.

Preferred embodiments are set out in the dependent claims and in the remaining part of the description.

In accordance with several embodiments, a device for delivering fluid (e.g., viscoelastic, surgical fluid, targeted drug, chemical, solution, medicament, or other liquid or gas) includes a housing (e.g., proximal housing), the housing including a fillable reservoir for storing fluid and a connector configured to be fluidically coupled to a pre-filled fluid canister or vial (e.g., ophthalmic viscoelastic device or syringe). The device further includes a cannula having a proximal end, a distal end, and a lumen, the proximal end of the cannula coupled to a distal end of the housing, the distal end of the cannula configured to penetrate an eye of a patient via a corneal incision, and the lumen of the cannula extending from the proximal end to the distal end of the cannula. The device also includes a catheter having a proximal end, a distal end, and a lumen, the proximal end of the catheter fluidically coupled to the reservoir, and the lumen extending from the proximal end of the catheter to the distal end of the catheter.

The fluid delivery device may also include a first actuator configured to, when actuated, advance the catheter along the lumen of the cannula and cause the catheter to exit the distal end of the cannula. The fluid delivery device may further include a second actuator configured to, when actuated, cause the fluid stored in the reservoir to flow through the catheter and exit the distal end of the catheter. The lumen of the catheter is configured to allow the fluid stored in the reservoir to flow through the catheter and into the eye of the patient. The catheter is sized so as to extend out of the distal end of the cannula and along an entire 360-degree circumference of Schlemm's canal of the patient when the first actuator is actuated.

In some implementations, the distal end of the cannula comprises a scoop with a sharpened tip. The distal end portion of the cannula may be pre-curved or angled. At least a distal portion of the catheter may be pre-shaped to approximately follow or match with a radius of curvature of Schlemm's canal.

In some implementations, at least a distal portion of the catheter is flexible. In some implementations, the cannula comprises rigid material.

In some implementations, the distal end of the cannula includes one or more cutouts (e.g., one cutout, two adjacent cutouts) and/or two or more anchors to form a cutting tip or surface. In some implementations, the distal end of the cannula includes a beveled tip.

The housing may further include a channel (e.g., sliding channel). The first actuator may comprise a first sliding trigger adapted to translate axially within the channel between a proximal position and a distal position and the second actuator may comprise a second sliding trigger adapted to translate axially within the channel between a proximal position and a distal position. When the first sliding trigger is in the proximal position, the distal end of the catheter is positioned between the proximal end and the distal end of the cannula. When the first sliding trigger is in the distal position, the distal end of the catheter is advanced beyond the distal end of the cannula. Movement (e.g., axial movement) of the second sliding trigger between the proximal position and the distal position may cause a predetermined amount of the fluid to be dispensed via the distal end of the catheter. In some implementations, an amount of axial movement of the second sliding trigger along the channel corresponds to amount of the fluid dispensed from the catheter.

In some configurations, the catheter is positioned and sized such that the distal end of the catheter is adapted to be aligned with the distal end of the cannula when the first actuator is retracted to a proximal-most position within the channel. The first actuator and the second actuator are configured to be actuatable by a single hand of a single operator or user.

In accordance with several embodiments, a device configured to deliver fluid (e.g., viscoelastic) to at least one ophthalmic drainage passage (e.g., Schlemm's canal, collector channels, episcleral venous system, subretinal space, subscleral space) includes a housing including a proximal connector and a reservoir, the reservoir configured to store fluid, the proximal connector configured to fluidically couple to a pre-filled ophthalmic viscoelastic device or other fluid source. The device further includes a cannula configured to be inserted into Schlemm's canal through a corneal incision, the cannula including a lumen. The device also includes a catheter having a proximal end, a distal end, and a lumen, the proximal end fluidically coupled to the reservoir, the distal end configured to advance past a distal end of the cannula, the lumen configured to allow the fluid stored in the reservoir to flow through the catheter and be dispensed via the distal end of the catheter.

The device further includes a first slidable trigger configured to move between an unactuated position and an actuated position (e.g., via a finger or thumb) to cause the catheter to advance along the lumen of the cannula and past the distal end of the cannula. The device also includes a second slidable trigger configured to move between an unactuated position and an actuated position to cause the fluid stored in the reservoir to be dispensed into Schlemm's canal via the lumen of the catheter.

In accordance with several embodiments, a device configured to deliver fluid to at least one ophthalmic drainage passage includes a reservoir configured to store fluid, a cannula comprising a lumen and configured to penetrate an eye of a patient via a corneal incision, and a catheter configured to be advanced along the lumen of the cannula. The catheter includes a lumen configured to allow the fluid stored in the reservoir to be dispensed via the catheter. The device also includes a first actuator and a second actuator, wherein actuation of the first actuator causes the catheter to advance along the cannula, and wherein actuation of the second actuator causes the fluid to be dispensed from the reservoir through the catheter.

In some implementations, the second actuator is configured to slide between an unactuated position and an actuated position to cause the fluid to be dispensed. An amount of the fluid dispensed is based at least in part on distance between the unactuated position and the actuated position.

In some implementations, the catheter comprises a bulbed distal tip. At least a portion of the catheter may include a phosphorescent colorant. At least a portion of the catheter may include contrast marks spaced along a length of the catheter. At least the distal end of the cannula may be comprised of shape memory material. In some implementations, at least the distal end of the cannula includes notches to facilitate articulation of the distal end of the cannula.

In accordance with several embodiments, a cannula adapter configured for use with a pre-filled ophthalmic viscoelastic device to deliver viscoelastic to up to a full 360-degree circumference of Schlemm's canal in a single pass through a single entry point into Schlemm's canal includes a proximal housing, the proximal housing including a sliding channel therein and a connector configured to be axially translated back and forth within the sliding channel. A proximal end of the connector is configured to be fluidically coupled to the pre-filled ophthalmic viscoelastic device. The adapter further includes an outer cannula including a proximal end coupled to a distal end of the housing, a distal end configured to be inserted into Schlemm's canal through a corneal incision, and an elongate cannula portion including a lumen extending from the proximal end to the distal end of the outer cannula. The adapter includes an inner catheter including a proximal end fluidically coupled to a distal end of the connector, a blunt or rounded distal end configured to be advanced along at least a portion of a circumference of Schlemm's canal, and an elongate catheter portion including a lumen extending from the proximal end of the inner catheter to the distal end of the inner catheter. The inner catheter is sized to fit within and be advanced along the lumen of the outer cannula. The lumen of the inner catheter is configured to deliver viscoelastic from the ophthalmic viscoelastic device to Schlemm's canal. The inner catheter is sized so as to extend out of the distal end of the outer cannula and along an entire 360-degree circumference of Schlemm's canal when the connector is advanced to a distal-most position within the sliding channel.

In some implementations, the distal end of the outer cannula includes a scoop with a sharpened tip. In some implementations, the distal end of the outer cannula is pre-curved. At least a distal portion of the inner catheter may be pre-shaped to follow approximately a radius of curvature of Schlemm's canal. At least a distal portion of the inner catheter may be flexible. The outer cannula may comprise rigid material. The inner catheter may be positioned and sized such that the distal end of the inner catheter is adapted to be aligned with the distal end of the outer cannula when the connector is retracted to a proximal-most position within the sliding channel.

In some implementations, the cannula adapter further includes a fluid control adapter configured to be attached to a proximal end of the pre-filled ophthalmic viscoelastic device. The cannula adapter may further includes a sliding trigger mechanically coupled to the connector and adapted to translate axially relative to the housing.

In accordance with several embodiments, a cannula adapter configured for use with a pre-filled ophthalmic viscoelastic device to deliver viscoelastic to at least one ophthalmic drainage passage includes a proximal housing, the proximal housing including a sliding channel therein and a connector configured to be axially translated back and forth within the sliding channel. A proximal end of the connector is configured to be fluidically coupled to the pre-filled ophthalmic viscoelastic device. The cannula adapter further includes an outer cannula having a proximal end coupled to a distal end of the housing, a distal end configured to be inserted into Schlemm's canal through a corneal incision, and an elongate cannula portion including a lumen extending from the proximal end to the distal end of the outer cannula. The cannula adapter also includes an inner catheter including a proximal end fluidically coupled to a distal end of the connector, a distal end configured to be advanced along at least a portion of a circumference of Schlemm's canal, and an elongate catheter portion including a lumen extending from the proximal end of the inner catheter to the distal end of the inner catheter. The lumen of the inner catheter is configured to deliver viscoelastic from the ophthalmic viscoelastic device to Schlemm's canal.

In accordance with several embodiments, a cannula adapter configured to deliver viscoelastic to at least one ophthalmic drainage passage includes a proximal housing, the proximal housing comprising a rotatable dial and an actuator. The cannula adapter further includes an outer cannula including a proximal end mechanically coupled to the proximal housing, a distal end configured to be inserted into Schlemm's canal through a corneal incision, and an elongate cannula portion including a lumen extending from the proximal end to the distal end of the outer cannula. The cannula adapter also includes an inner catheter having a proximal end operatively coupled to the rotatable dial, a distal end configured to be advanced along at least a portion of a circumference of Schlemm's canal, and an elongate catheter portion including a lumen extending from the proximal end of the inner catheter to the distal end of the inner catheter. The elongate catheter portion of the inner catheter is positioned to fit within, and advance along, the outer cannula. The adapter is configured such that rotation of the dial causes advancement of the inner catheter along the outer cannula and then into and along up to a 360-degree circumference of Schlemm's canal. The proximal end of the inner catheter is fluidically coupled to a fluid dispensing mechanism. Operator actuation of the actuator allows fluid to be dispensed from a fluid reservoir of the fluid dispensing mechanism through the inner catheter and into Schlemm's canal.

In some implementations, the actuator includes a button adapted to be pressed by a finger or thumb of the operator and the dial is adapted to be rotated by the thumb or finger of the operator while the adapter is held in a single hand of the operator. The reservoir may be fillable with any amount and any type of fluid.

The fluid dispensing mechanism may include a spring driven system. The fluid dispensing mechanism may comprise an elastic or compliant tube adapted to be charged and discharged. In some implementations, the fluid dispensing mechanism comprises a peristaltic pump. The inner catheter may include a bulbed distal tip. At least a portion of the inner catheter may include a phosphorescent colorant. At least a portion of the inner catheter may include contrast marks spaced along a length of the inner catheter. At least the distal end of the outer cannula may be comprised of shape memory material. At least the distal end of the outer cannula may include notches to facilitate articulation of the distal end of the outer cannula.

### BRIEF DESCRIPTION OF THE DRAWINGS

**FIGURES 1A and 1B** illustrate top views of an embodiment of a cannula adapter in an un-deployed configuration and a fully deployed configuration, respectively.
**FIGURES 1C and 1D** illustrates an example of how the cannula adapter of FIGURE 1A is attached to a standard ophthalmic viscosurgical device, such as an ophthalmic viscoelastic syringe.
**FIGURES 1E and 1F** illustrate an example of a surgical implementation of access and insertion of the cannula adapter of FIGURE 1A.
**FIGURES 1G and 1H** illustrate another example of a surgical implementation of access and insertion of the cannula adapter of FIGURE 1A.
**FIGURE II** illustrates a top view of a system that includes the cannula adapter of FIGURE 1A in combination with an additional adapter coupled to a proximal end of the standard ophthalmic viscosurgical device of FIGURE 1C to facilitate controlled fluid delivery.
**FIGURES 2A and 2B** illustrate side views of another embodiment of a cannula adapter with a sliding trigger in both an un-deployed configuration and a fully-deployed configuration.
**FIGURE 2C** illustrates a side view of an adaptation of the embodiment of FIGURE 2A that includes a coupler to facilitate pneumatic fluid delivery.
**FIGURES 2D and 2E** illustrate an example embodiment of a pneumatic system adapted for use with the cannula adapter of FIGURE 2C.
**FIGURE 3A** illustrates another embodiment of a cannula adapter that utilizes a compressed spring to hold a force to the ophthalmic viscosurgical device.
**FIGURES 3B, 3C, and 3D** schematically illustrate an example operation scheme of the cannula adapter of FIGURE 3A.
**FIGURES 4A, 4B, and 4C** schematically illustrate a three-layer assembly that can be incorporated into any of the embodiments of the cannula adapters.
**FIGURE 4D** is a schematic, close-up, side view of a distal tip of the three-layer assembly of FIGURE 4C.
**FIGURE 4E, 4F, and 4G** schematically illustrate an example operation scheme of the three-layer assembly of FIGURES 4A, 4B, and 4C.
**FIGURES 5A-8B** schematically illustrate various embodiments of an internal support mechanism that may be incorporated into any of the embodiments of the cannula adapters.
**FIGURES 9A and 9B** schematically illustrate external top views of an embodiment of a cannula adaptor with a dial-feed system, showing an example operation scheme of the cannula adaptor.
**FIGURES 9C and 9D** schematically illustrate internal top views showing an example operation scheme of the cannula adaptor of FIGURE 9A.
**FIGURES 9E and 9F** schematically illustrate side views showing an example operation scheme of the cannula adaptor of FIGURE 9A.
**FIGURES 10A, 10B, and 10C** schematically illustrate an example operation scheme of a cannula adapter with a spring driven system having a fillable reservoir.
**FIGURES 11A and 11B** schematically illustrate an example operation scheme of a fillable cannula adapter having a threaded plunger fluid delivery system with reverse flow.
**FIGURES 12A and 12B** schematically illustrate an example operation scheme of an embodiment of a charged elastic tube fluid delivery mechanism.
**FIGURES 13A, 13B, and 13C** schematically illustrate an embodiment of a peristaltic fluid delivery mechanism.
**FIGURE 14** illustrates various examples of inner catheter shapes and features to facilitate visualization that may be incorporated into any of the embodiments of the cannula adapters.
**FIGURES 15A and 15B** schematically illustrate an example operation of a shape set cannula that may be incorporated into any of the embodiments of the cannula adapters.
**FIGURE 16** schematically illustrates an embodiment of a cannula having a notched distal tip to facilitate articulation that may be incorporated into any of the embodiments of the cannula adapters.
**FIGURE 17A** illustrates a perspective view of another embodiment of a cannula adapter, or fluid delivery device.
**FIGURE 17B** illustrates a perspective, exploded view of the cannula adapter of FIGURE 17A.
**FIGURES 17C and 17D** illustrate cross-sectional views of the cannula adapter of FIGURE 17A.
**FIGURE 17E** illustrates an enlarged, cross-sectional view of the cannula adapter of FIGURE 17A as indicated in FIGURE 17D.
**FIGURE 17F** illustrates an enlarged, cross-sectional view of a tip of the cannula adapter of FIGURE 17A.
**FIGURE 17G** illustrates a perspective view of the cannula adapter of FIGURE 17A without a locking pin.
**FIGURE 17H** illustrates a cross-sectional view of the cannula adapter of FIGURE 17G.
**FIGURES 18A and 18B** illustrate various views of an outer cannula that may be incorporated into any of the embodiments of the cannula adapters.
**FIGURES 18C and 18D** illustrate various views of an inner catheter that may be incorporated into any of the embodiments of the cannula adapters.
**FIGURES 18E, 18F, 18G, and 18H** illustrate various views of examples of a distal tip of a cannula of a fluid delivery device.
**FIGURES 19A and 19B** illustrate an example operation scheme of another embodiment of a cannula adapter.

### DETAILED DESCRIPTION

Described and illustrated herein are various embodiments of systems, devices and methods to facilitate delivery of fluid (e.g., viscoelastic fluid, drugs in liquid or fluid form, chemicals, solutions) to (e.g., within) ophthalmic passages or spaces (e.g., Schlemm's canal, collector channels, downstream episcleral venous networks, tissue tracts of the eye, suprachoroidal space, subconjunctival space, subretinal space) within a mammalian eye (e.g., human eye or eye of other mammals, such as monkeys or horses). For example, the ophthalmic passages or spaces may be adapted to facilitate drainage of aqueous humor to control (e.g., reduce) intraocular pressure. The control of intraocular pressure may treat or reduce symptoms associated with glaucoma or other ophthalmic disorders or conditions.

In accordance with several embodiments, the systems, devices and methods described and illustrated herein provide a simple, controlled, and potentially low-cost solution to delivery of viscoelastic or other fluid to up to 360 degrees of Schlemm's canal in one pass, or one single traverse along the canal, and through a single minimally-invasive incision (e.g., self-sealing corneal incision).

In accordance with several embodiments, the systems, devices and methods described and illustrated herein provide one or more of the following advantages or benefits: (i) lower cost than existing treatment options, (ii) improved ease-of-use that is less complex than existing treatment options; (iii) adapts to existing viscoelastic syringes or ophthalmic viscosurgical devices, preventing the need for a pre-filling step; (iv) improved performance because dispensing is decoupled from catheter motion, thereby allowing precise dispensing location and volume of fluid at any time during a procedure; (v) minimally-invasive, requiring only a single incision; (vi) configured to treat up to 360 degrees of Schlemm's canal with one pass; (vii) does not require extensive training or experience to use; (viii) patient-dependent and/or location-dependent flexibility in treatment; (ix) single-person operation to provide independent control of fluid dispensing and catheter motion; (x) single-hand operation; (xi) single sharp-tipped component; (xii) increased trackability of location of the inner catheter within Schlemm's canal; (xiii) accommodates any choice or operator discretion of viscoelastic or other fluid and different amounts of viscoelastic or other fluid; (xiv) controlled, or fixed volume, microbolus dispensing; and/or (xv) all-in-one integrated system instead of multiple separate instruments inserted separately.

In accordance with several embodiments, the systems and devices described herein provide improved performance compared to systems in which the amount of viscoelastic or other fluid is proportional to a return displacement of the delivery catheter, which often leads to underdelivery of viscoelastic or other fluid. Systems involving fixed delivery volume neglect the logical presumption that effective treatment volumes may be patient-dependent and location dependent. In addition, limiting delivery to return displacement prevents the operator (e.g., surgeon) from delivering fluid through potential obstructions during forward progression of the delivery catheter through tissue such as Schlemm's canal.

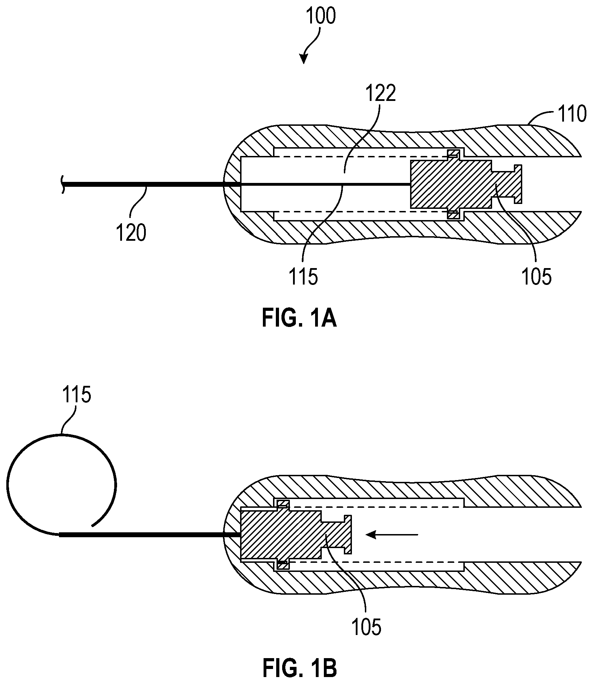

**FIGURES 1A and 1B** schematically illustrate top views of an embodiment of a cannula adapter 100 in an undeployed configuration and a deployed configuration, respectively. The cannula adapter 100 includes a connector 105, a housing 110, an inner catheter 115, and an outer cannula 120.

The connector 105 (e.g., standard Luer connector) may be adapted to slide along a restricted axial range within the housing 110 of the cannula adapter 100. The connector 105 may also be restricted from azimuthal rotation. The inner catheter 115 may comprise a flexible lumen that is introduced through Schlemm's canal and that is fluidically coupled to a distal end of the sliding connector 105. The lumen of the inner catheter 115 may extend from a proximal end to a distal end of the inner catheter 115. The inner catheter 115 may be adapted to fit within and extend through a lumen of the outer cannula 120 such that a distal tip of the inner catheter 115 and a distal tip of the outer cannula 120 are aligned when the sliding connector 105 is in a proximal-most position, such as in the undeployed configuration shown in FIGURE 1A.

The proximal end of the outer cannula 120 can be coupled to the distal end portion of the housing 110 (and to a distal end of a sliding channel 122 of the housing within which the connector 105 translates axially). When the connector 105 is in a distal-most position (such as in the fully-deployed configuration shown in the FIGURE 1B when the connector 105 has been pressed forward until the sliding channel 122 within the housing 110 ends), the distal tip of the inner catheter 115 can extend past the distal tip of the outer cannula 120 to a predetermined distance. In some implementations, the predetermined distance can be approximately equal to the circumference (e.g., 360 degrees) of Schlemm's canal. The length of the portion of the inner catheter 115 that is extended out of the distal tip of the outer cannula 120 when the connector 105 is in a distal-most position within the sliding channel 122 of the housing 110 may be configured to have a length corresponding to a value in an upper range (e.g., a maximum known value) of a circumference of Schlemm's canal of a human or other mammal or a length corresponding to a mean or median value.

The distal portion (e.g., at least the portion that extends past the including the distal tip) of the inner catheter 115 may be pre-shaped (e.g., shape set using shape memory material such as copper-aluminum-nickel alloy or nickel-titanium alloy) to follow approximately a radius of curvature of Schlemm's canal (e.g., a predetermined median or mean value), or may be made of material flexible enough to bend along an outer wall of Schlemm's canal (e.g., polyvinylchloride, polyetheretherketone, polyethylene, polytetrafluoroethylene, thermoplastic polyurethane, polyamide, polyimide, polymethyl methacrylate (PMMA), acrylonitrile butadiene styrene, silicone, and/or other sufficiently flexible material).

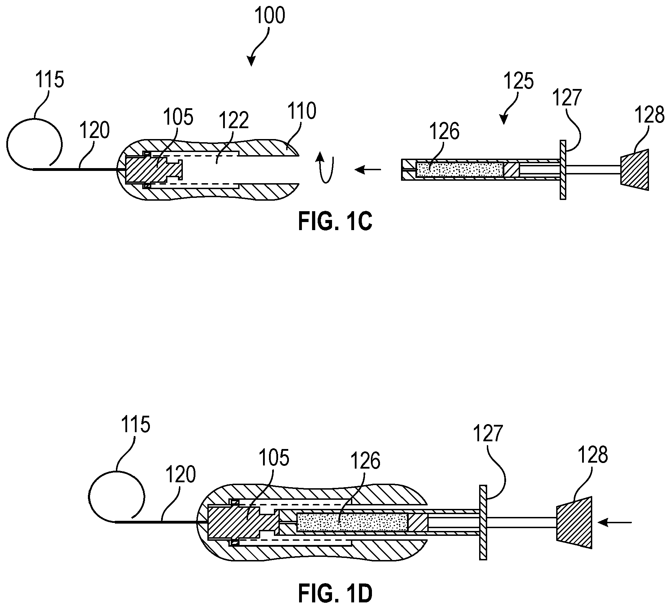

The cannula adapter 100 of FIGURES 1A and 1B may be adapted for use with any standard "off-the-shelf" or commercially-available ophthalmic viscosurgical device (OVD) or other viscoelastic delivery device, such as the HEALON^{®} viscoelastic syringes made available commercially by Johnson & Johnson. For example, a pre-filled ophthalmic viscoelastic device (e.g., syringe) 125 can be inserted into the proximal end of the housing 110 and rotated to rigidly lock with the sliding connector 105 as shown in **FIGURE 1C****.** As an example of use, an operator (e.g., clinician or medical practitioner) can hold the housing 110 with one hand and use the other hand to translate the syringe 125 forward (e.g., distally, or toward a patient) relative to the housing 110 (e.g., by pressing on a proximal stop 127 of the syringe 125) to extend the inner catheter 115 (e.g., via translation of the sliding connector 105) out of the outer cannula 120, and then plunge the syringe 125 (e.g., by pressing on a proximal plunger actuator 128 of the syringe 125 as shown in **FIGURE 1D****)** to deliver a desired amount of fluid 126 (e.g., viscoelastic or other ophthalmic viscosurgical device (OVD) fluid). The desired amount may be patient-specific or location specific and may be determined by the operator as desired and/or required.

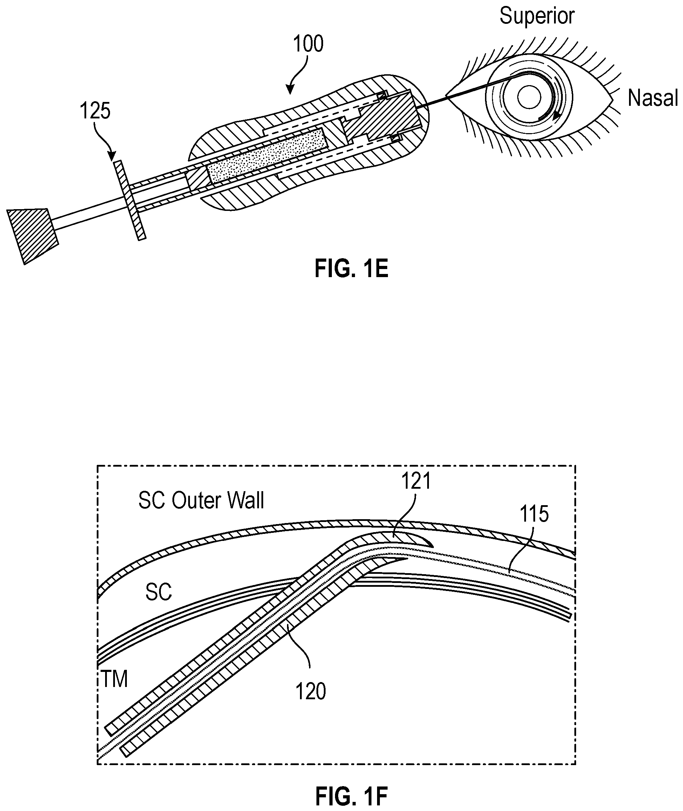

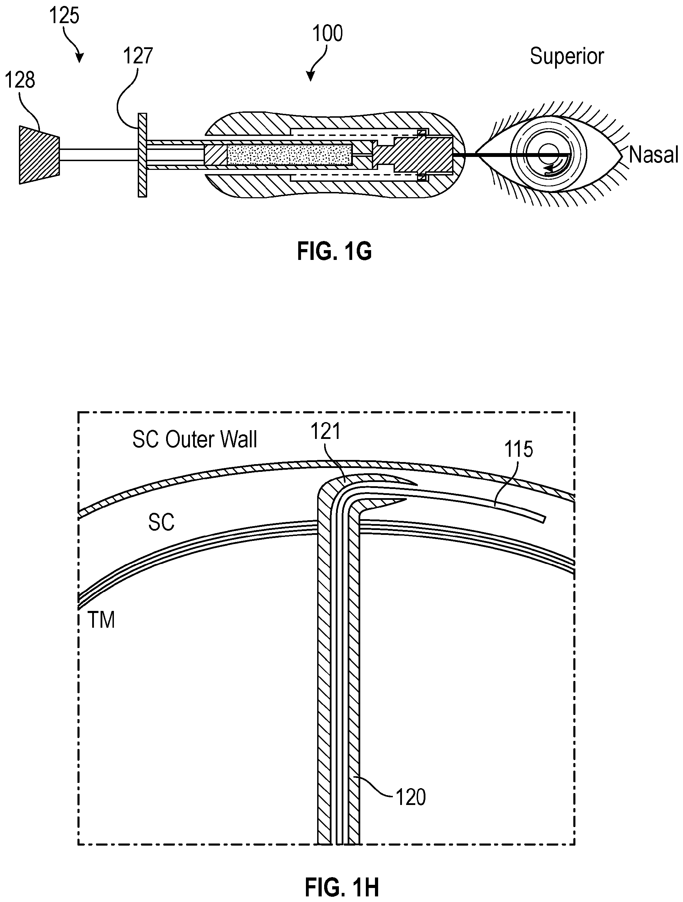

The outer cannula 120 can be adapted to be inserted through a minimally-invasive, temporal, clear corneal incision. The corneal incision may be sized so as to be self-sealing without requiring sutures. **FIGURE 1E** illustrates one example method of surgical insertion using a superior insertion approach. As illustrated, a distal portion 121 of the outer cannula 120 may be shaped (e.g., a scoop with a sharpened tip) so as to facilitate penetration of a superior trabecular meshwork (TM) portion and guide the inner catheter 115 360 degrees into Schlemm's canal (SC) as shown in **FIGURE 1F****.** Alternatively, the distal tip of the outer cannula 120 may be inserted using a nasal insertion approach through the nasal trabecular meshwork, as shown in **FIGURES 1G and 1H****.**

In some implementations, such as when using a nasal insertion approach, the inner catheter 115 may be adapted to be advanced through a first 180 degrees of Schlemm's canal and then through a remaining 180 degrees of Schlemm's canal. For example, a distal portion 121 of the outer cannula 120 may be inserted into Schlemm's canal through the trabecular meshwork in such a manner that the inner catheter 115 may be advanced through a first 180 degrees of Schlemm's canal from the insertion location into Schlemm's canal. The inner catheter 115 may then be retracted and the distal tip 121 of the outer cannula 120 may be rotated, or removed from Schlemm's canal and reinserted, in a manner such that the remaining 180 degrees of Schlemm's canal may be traversed by the inner catheter 115 in an opposite direction from the entry point. The distal tip 121 of the outer cannula 120 may be pre-curved or may be sufficiently flexible to bend upon contact with an outer wall of Schlemm's canal. If pre-curved, the distal tip of the outer cannula 120 may have a different curvature or bend configuration depending on whether it is intended for a superior or nasal insertion approach. In some implementations, only a single 180 degree portion is treated.

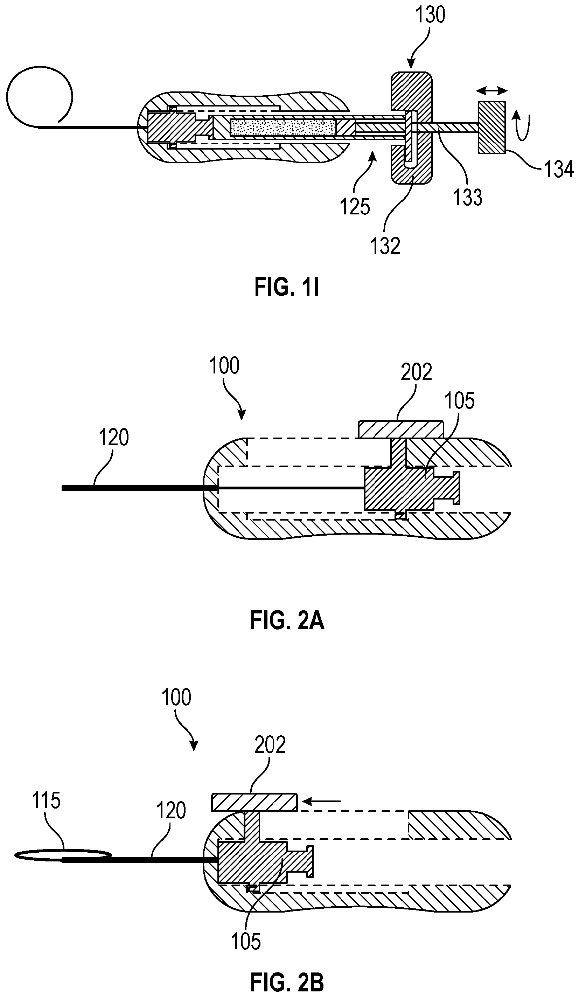

Turning to **FIGURE 1I****,** a fluid control adapter 130 can be attached to a proximal end of the syringe 125 to facilitate precisely controlled delivery of viscoelastic or other fluid (e.g., drug, chemical, solution, other liquid) in accordance with several embodiments. In such implementations, a plunger handle of the syringe 125 may be removed. In the illustrated embodiment, the fluid control adapter 130 comprises a dispenser attachment fitting 132 that is adapted to be attached to the syringe 125 like a common pneumatic adhesive dispenser (such as a pneumatic dispenser made commercially available by Nordson EFD). The attachment fitting 132 may have a threaded thru-hole for receiving a complementary threaded "bolt-type" plunger driver 133 and a rotatable knob 134. Rotating the knob 134 (and thereby the plunger driver 133) clockwise can push the internal plunger of the syringe 125, thus dispensing the fluid 126 (e.g., viscoelastic or other OVD fluid). The sensitivity of the dispensing can be controlled by the pitch of the threading. The fluid control adapter 130 can be rigidly connected to the syringe 125 and therefore axial translation of the adapter 130 (e.g., caused by axial translation of the knob 134 from rotation of the knob 134) can result in extension of the inner catheter 115. The rotatable knob 134 may optionally comprise a knurled head.

With reference to **FIGURES 2A and 2B****,** a cannula adapter 200 may be adapted or modified to include a sliding trigger 202 that is rigidly attached to the internal sliding connector 105 (e.g., a single molded part during manufacturing), and allowed to slide axially relative to the housing 110. The operator can then simply extend the inner catheter 115 via distal motion (i.e., motion toward a patient) of his or her finger or thumb instead of pressing on a proximal stop of the syringe 125 (e.g., pressing on a proximal plunger actuator 128 of the syringe 125 as shown in **FIGURE 1D****).** An outer surface of the sliding trigger 135 may have surface features (e.g., ridges, grooves, or the like) adapted to facilitate gripping or friction against a finger or thumb of the operator.

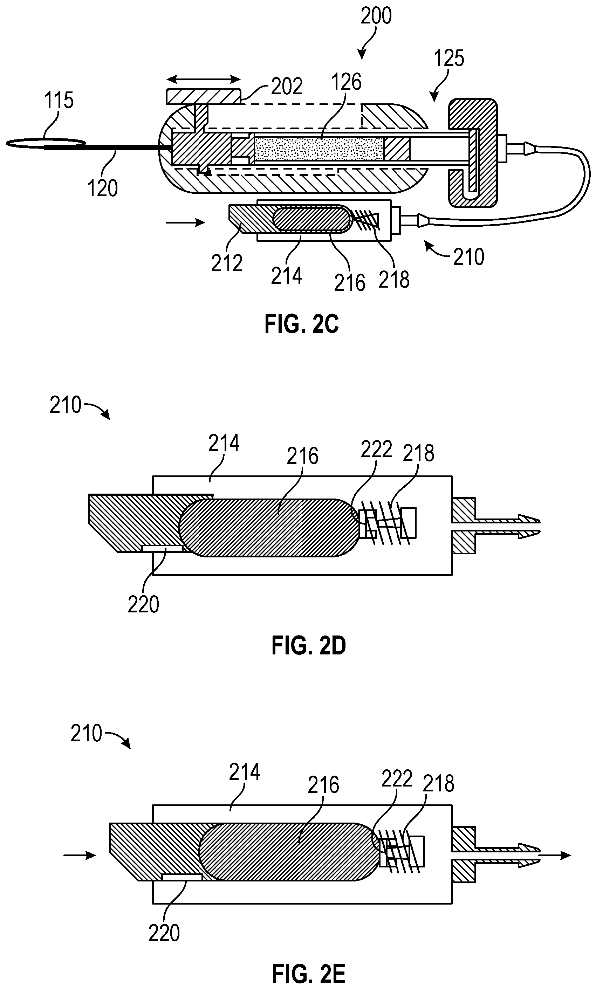

**FIGURE 2C** illustrates a further modification or adaptation of the cannula adapter 200 of FIGURES 2A and 2B that is adapted to allow for actuation of the inner catheter 115 and dispensing of the viscoelastic or other fluid with a single hand of the operator. The illustrated embodiment can include the sliding trigger 202 introduced in FIGURES 2A and 2B to extend the inner catheter 115 and incorporates a pneumatic system 210 adapted for pressurizing the viscoelastic syringe 125. The pneumatic system 210 is enlarged and illustrated in more detail with respect to its components and operation in **FIGURES 2D and 2E****.**

The pneumatic system 210 can utilize a trigger 212 that is adapted to be actuated by an operator's finger (e.g., index finger). FIGURE 2D illustrates the pneumatic system 210 before the trigger 212 is depressed and FIGURE 2E illustrates the pneumatic system 210 after the trigger is depressed (e.g., pushed in a direction away from the patient to compress the spring 218). Upon depression of the trigger 212 (e.g., moves to its actuated position), a miniature, valve-release pressurized cartridge 216 (e.g., a pico cylinder made commercially available by Picocyl) can open to pressurize a chamber 214 that is fluidically coupled to a proximal side of the syringe plunger (e.g., using an attachment similar to a common pneumatic adhesive dispenser). This arrangement or mechanism can advantageously allow for a steady, controlled dispensing of a pressurized supply of the fluid 126 (e.g., viscoelastic or other OVD fluid) from the syringe 125. When the trigger 212 is released, a spring 218 can relax to an uncompressed configuration and pushes the pressure cartridge 216 back to its nominal location and the chamber 214 is vented via a cutout 220 to, for example, abruptly stop viscoelastic or other fluid dispensing. As shown in FIGURE 2E, a valve 222 of the pressure cartridge 216 may be open and the spring 218 compressed when the trigger 142 is depressed, and the valve 222 may be closed and the spring 218 relaxed or extended (e.g., moves back to its unactuated position) when the trigger 212 is released.

The trigger 212 can include a cutout 220 formed on a side of its body. Before the trigger 212 is depressed, the cutout 220 may not be fluidly connected with the chamber 214. As the trigger 212 is depressed, the cutout 220 can create a path between the chamber 214 and the ambient. When the trigger 212 is fully compressed and the cartridge 216 opens to pressurize the chamber 214, the cutout 220 may be separated from the ambient so that pressure in the chamber 214 may be retained. When the trigger 212 is released and moves back to its unactuated position, the cutout 220 can create a path between the chamber 214 and the ambient to vent the chamber 214 to, for example, as described herein, abruptly stop viscoelastic or other fluid dispensing.

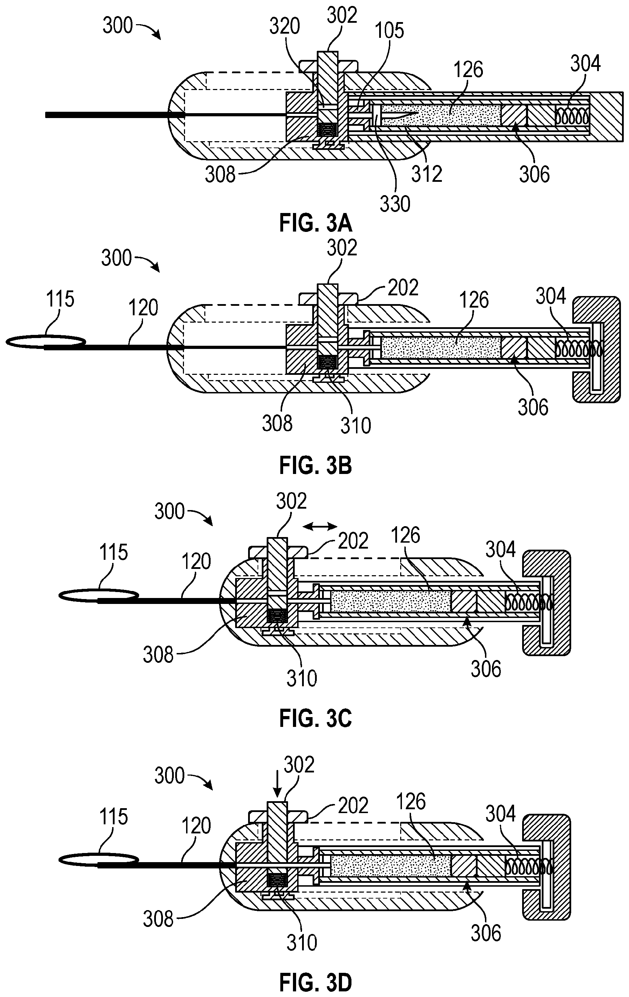

**FIGURES 3A-3D** illustrate alternative embodiments of a viscoelastic dispensing mechanism incorporating valves and springs that may be incorporated into any of the embodiments described herein. FIGURE 3A shows a cannula adapter 300 that utilizes a compressed spring 304 to hold a force to a viscoelastic cartridge/plunger 306. As described herein, actuating the (e.g., sliding towards the patient) the sliding trigger 202 can cause the catheter 115 to be extended out from a distal end of the outer cannula 120. In the example illustrated in FIGURE 3A, the connector 105 can include a dispense button 302 and a valve 308. The dispense button 302 can have an unactuated position and an actuated position. When in the unactuated position (as shown in FIGURE 3C), the body of the dispense button 302 can break the flow path within the connector 105 and, for example, prevent the fluid 126 from flowing from the viscoelastic cartridge 306 into the catheter 115. When in the actuated position (e.g., pressed downwards to compress a spring 310 as shown in FIGURE 3D), a lumen 320 formed within the dispense button 302 can connect with the flow path within the connector 105 and between the viscoelastic cartridge 306 and the catheter 115. When the dispense button 302 is released, the spring 310 can urge the dispense button 302 to return to the unactuated position and break the flow path between the viscoelastic cartridge 306 and the catheter 115.

In some implementations, as shown in FIGURE 3A, the viscoelastic cartridge 306 includes an optional diaphragm 330, and the connector 105 (e.g., Luer connector as shown in FIGURE 1A) can include a hypodermic needle 312 to puncture the diaphragm 330.

The viscoelastic cartridge 306 may be housed in a single sliding assembly (as shown in FIGURE 3A) or may have a separate attachment with a plunger spring 304 (as shown in FIGURES 3B-3D). With reference to FIGURES 3B-3D, which better illustrates operation of the viscoelastic dispensing mechanism, the thumb slider/trigger 202 can be used to actuate the inner catheter 115 in and out of Schlemm's canal (as shown in FIGURES 3C and 3D), while the dispensing button 302 is used to dispense the fluid 126 (e.g., viscoelastic or other OVD fluid) from the viscoelastic cartridge 306 (as shown in FIGURE 3D).

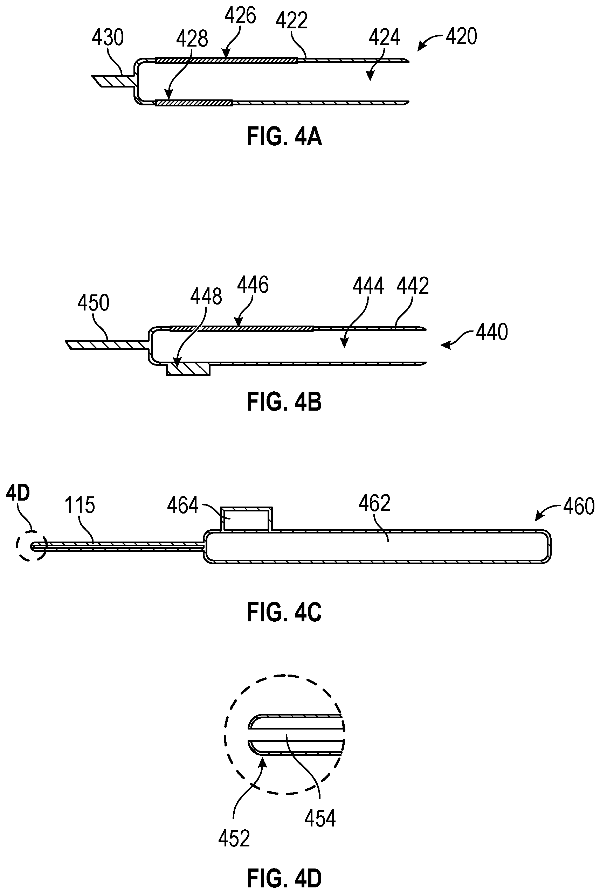

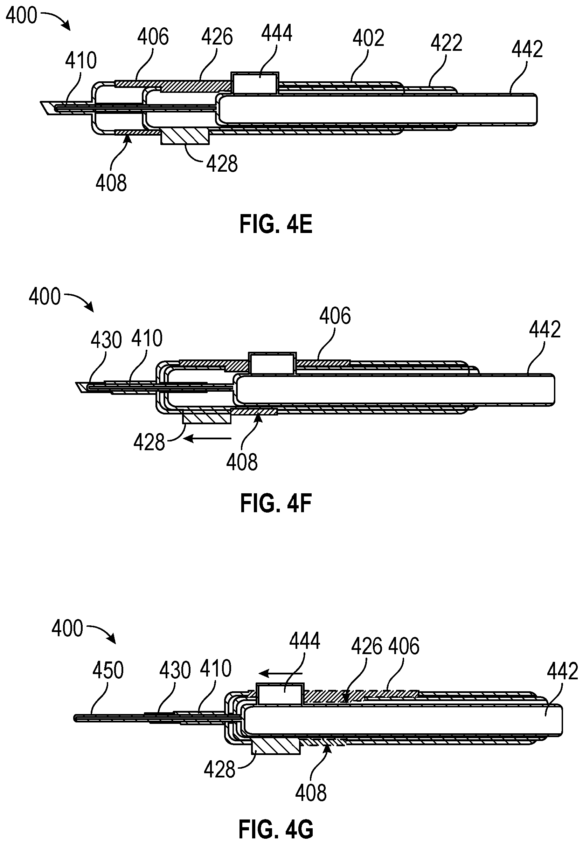

In accordance with several embodiments, the cannula adapters described and illustrated herein may be configured with multiple sliding layers as shown schematically in **FIGURES 4A-4C****.** The multiple sliding layers may comprise three layers: a needle assembly 420 comprising a sharp distal tip designed for penetration through the cornea, a cannula assembly 440 comprising a tip designed to traverse the anterior chamber and penetrate the trabecular meshwork, and an inner catheter assembly 460 with a blunt or rounded distal tip designed to traverse Schlemm's canal and a lumen to deliver viscoelastic or other fluid. FIGURE 4A illustrates the needle assembly 420 of a cannula adapter 400 (as shown in FIGURE 4E), FIGURE 4B illustrates the cannula assembly 440, and FIGURE 4C illustrates the inner catheter assembly 460.

The needle assembly 420 and the cannula assembly 440 may include slots as illustrated. The needle assembly 420 can include a body 422, a lumen 404, an upper slot 426, a lower slot 428, and a needle 430. The needle 430 may be integrated with the body 422. The slots 426, 428 can be formed opposite from each other on the body 422. In some implementations, the locations of the slots 426, 428 can be different from the example illustrated in FIGURE 4A. The cannula adapter 440 can includes a body 442, a lumen 444, an upper slot 446, a cannula slider 448, and a tip 450. The tip 450 and the cannula slider 448 can be integrated with the body 442 such that movement of the cannula slider 448 can translate to movement of the body 442 and the tip 450. The upper slot 446 may be formed on the body 442. The inner catheter assembly 460 can include a body 462, a catheter slider 464, and the catheter 115. The catheter slider 464 and the catheter 115 may be integrated with the body 462 such that movement of the catheter slider 464 can translate to movement of the body 462 and the catheter 115.

The operation, and assembled arrangement, of the three sliding layers of the cannula adapter 400 is shown schematically in **FIGURES 4E-4G****.** The cannula adapter 440 can be positioned within the needle assembly 420 such that the upper slot 446 of the cannula adapter 440 overlaps with at least a portion of the upper slot 426 of the needle assembly 420 and the cannula slider 448 is positioned within the lower slot 428 of the needle assembly 420. The catheter assembly 460 can be positioned within the cannula assembly 440 such that the catheter slider 464 is positioned within the upper slots 426, 446 of the needle assembly 420 and the cannula assembly 440, respectively.

The needle 430 can, as described herein, penetrate cornea of a patient. The cannula slider 448 can slide within the slot 428 the needle assembly 420 to cause the cannula assembly 440 to slide within the needle assembly 420. When the cannula slider 448 is moved distally (e.g., towards the patient), the tip 450 of the cannula assembly 440 can move distally and, for example, penetrate a superior trabecular meshwork (TM) portion of the patient. The slot 446 of the cannula adapter 440 can overlap with at least a portion of the slot 426 of the needle assembly 420 to allow the catheter slider 464 to slide distally (e.g., towards the patient) and proximally (e.g., away from the patient) within the slots 426, 446. When the catheter slider 444 is moved distally (e.g., towards the patient), the catheter 115 can, for example, exit via a distal end of the tip 450 and, for example, enter and travel along the Schlemm's canal. **FIGURE 4D** shows a close-up side view of a distal tip of the catheter 115. As shown in FIGURE 4D, the catheter 115 can include a rounded tip 452 and a lumen 454 that allows viscoelastic fluid or other fluid to flow through.

The three sliding layer assembly shown and described in FIGURES 4A-4G may advantageously remove the need for multiple separate surgical instruments, thereby streamlining the surgery.

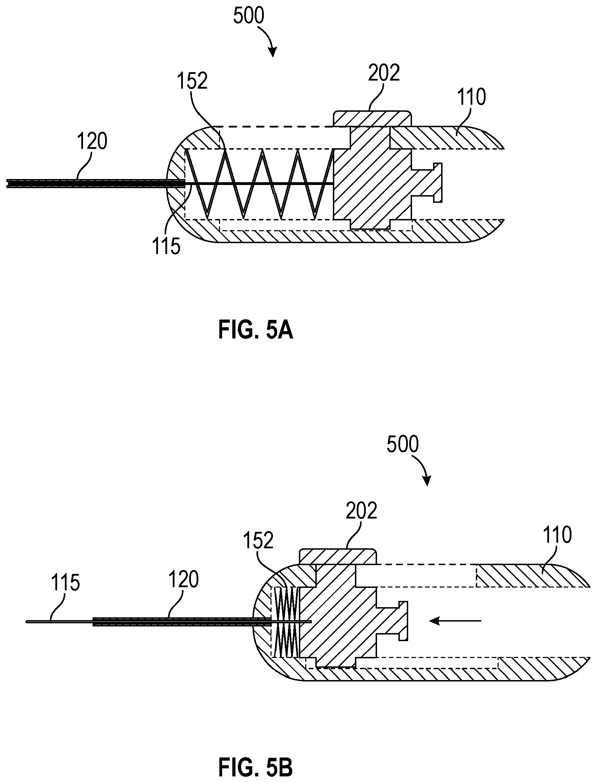

**FIGURES 5A-8B** illustrate internal components of various embodiments of the cannula adapters described herein. A clear challenge with larger catheter extension is prevention of internal buckling as the inner catheter 115 is fed through the outer cannula 120. **FIGURES 5A-8B** show various internal support mechanisms (that may be used separately or one or more mechanisms may be combined together) designed to prevent buckling and guide the inner catheter 115 along the outer cannula 120.

**FIGURES 5A and 5B** illustrate side views (undeployed configuration and fully-deployed configuration, respectively) of a cannula adapter 500 including a collapsible, helical support 152 with an inner diameter that is just larger than the inner catheter 115 and an outer diameter confined by the housing 110. The helical support 152 can have a near-zero spring force, but still maintain radial stability.

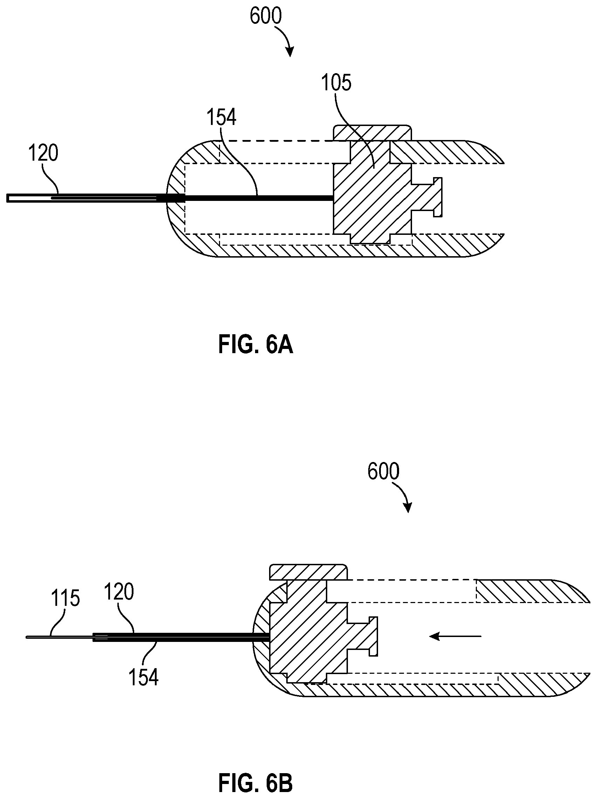

**FIGURES 6A and 6B** illustrate side views (undeployed configuration and fully-deployed configuration, respectively) of a cannula adapter 600 including a rigid support tube 154 with an inner diameter sized to fit the inner catheter 115 and an outer diameter sized to slide within the outer cannula 120. The rigid support tube 154 may be formed of any suitable rigid material (such as a 300 series stainless steel). In some implementations, use of the rigid support tube 154 may cause an increase in the diameter of the outer cannula 120.

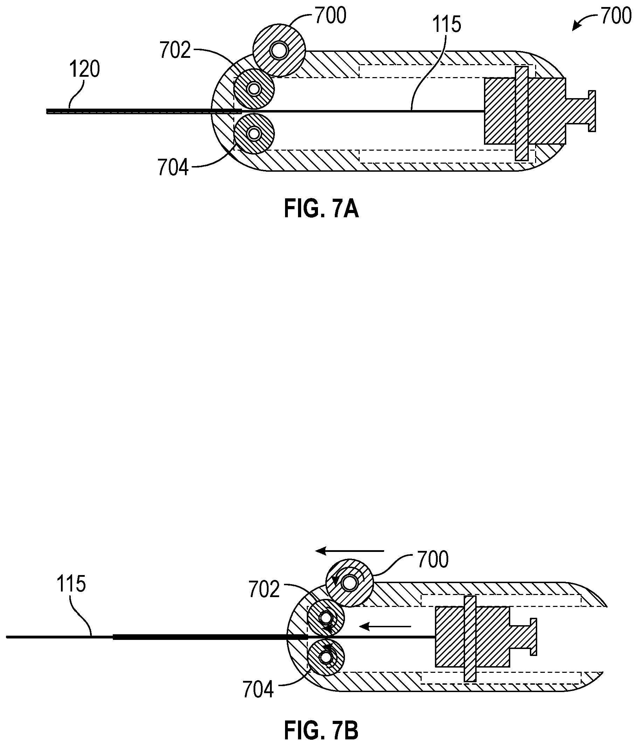

**FIGURES 7A and 7B** illustrate side views of a cannula adapter 700 including a wheel 701 and catheter feeders 702, 704. The catheter feeders 702, 704 can stabilize and prevent bending of the catheter 115 as it moves distally (e.g., towards the patient) or proximally (e.g., away from the patient). The wheel 701 can be coupled to the catheter feeder 702 such that rotation of the wheel 701 can translate to rotation of the catheter feeder 702. For example, as shown in FIGURE 7B, counterclockwise rotation of the wheel 701 can cause clockwise rotation of the catheter feeder 702. The clockwise rotation of the catheter feeder 702 can cause the catheter 115 to traverse distally (e.g., towards the patient). Likewise, clockwise rotation of the wheel 701 can cause counterclockwise rotation of the catheter feeder 702, which can cause the catheter 115 to traverse proximally (e.g., away from the patient). In some implementations, the catheter feeders 702, 704 are coupled to each other such that rotation of one causes rotation of another. For example, clockwise rotation of the catheter feeder 702 can cause counterclockwise rotation of the catheter feeder 704 (as shown in FIGURE. 7B). In some implementations, the movement (e.g., distal or proximal) of the catheter 115 can cause rotation of the catheter feeder 704. In some implementations, the catheter 115 can be retracted by simply pulling on the assembly from the syringe 125 (e.g., instead of rolling the wheel 700 in clockwise direction).

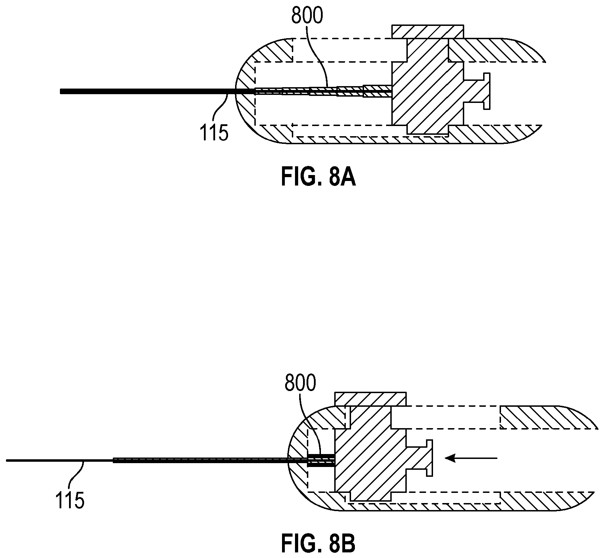

**FIGURES 8A and 8B** illustrate side views (e.g., undeployed configuration and fully-deployed configuration, respectively) of a telescoping tube 158 that provides support for the catheter 115 while collapsing during actuation, which may be incorporated into any of the embodiments of cannula adapters or fluid (e.g., viscoelastic) delivery devices described or illustrated herein.

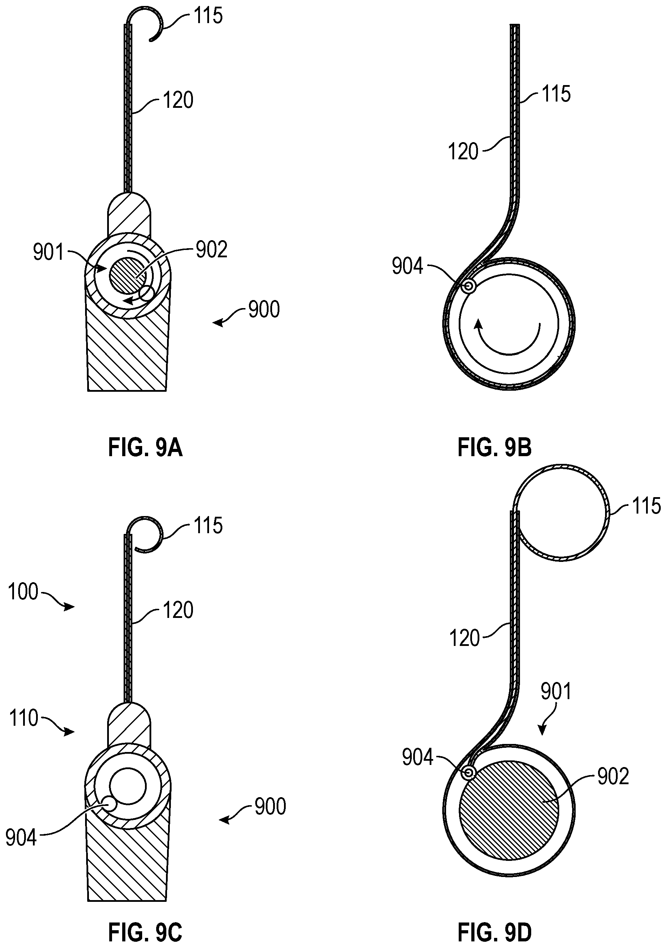

**FIGURES 9A-9F** illustrate a feed mechanism 900 that may be used to feed the catheter 115 into the outer cannula 120. The feed mechanism 900 may be incorporated into any of the embodiments of cannula adapters described or illustrated herein. The feed mechanism 900 can include a thumb-controlled dial 901 positioned near a distal end of the housing 110 to facilitate single-handed operation by an operator.

The dial 901 can be rotated by a thumb of the operator by interaction with a nub or protrusion 904 extending upward from the dial 901. Alternatively, the nub or protrusion 904 could include a dimple or indent. The dial 901 can be operatively coupled to a proximal end of the inner catheter 115 such that rotation of the dial 901, for example, in a clockwise direction advances the inner catheter 115 along and then out of the outer cannula 120 and into and along Schlemm's canal in a controlled manner, and rotation of the dial 901, for example, in a counter-clockwise direction retracts the inner catheter 115. Of course, these directions could be reversed as desired and/or required. The distal portion of the inner catheter 115 that is adapted to extend out of the distal tip of the outer cannula 120 upon full rotation of the dial 901 (e.g., the internal loop) may have a length adapted to match or correspond to (e.g., is approximately equal to) a full 360-degree circumference of Schlemm's canal as described herein. In some implementations, 360-degree rotation of the dial 901 (as shown in FIGURES. 9B and 9D) corresponds to 360 degrees of travel along Schlemm's canal by the catheter 115. In addition, the distal portion of the inner catheter 115 may include pre-shaped (e.g., shape set material with a defined radius of curvature corresponding to the radius of curvature of Schlemm's canal) or flexible material features as described herein.

The dial 901 can include a button 902 that can be depressed by the thumb of the operator. The button 902, when actuated (e.g., move from an unactuated position to an actuated position) may cause dispensing of viscoelastic or other viscosurgical fluid. For example, the button 902, when actuated, can cause (e.g., actuate) a control valve to dispense the viscoelastic or other viscosurgical fluid. Dispensing of the viscoelastic or other viscosurgical fluid may occur at a constant rate, a variable rate based on an amount that the button 902 is depressed, or a predetermined bolus amount per button press. In some implementations, actuation of the button 902 may actuate a pump such as a positive displacement pump.

In some implementations, the feed mechanism 900 may include indents or detents positioned at clock hours around the dial 901. The indents or detents can provide the operator an indirect indication of the azimuthal position of the catheter 115 within Schlemm's canal without requiring actual visualization via imaging modalities. For example, the detents at each clock hour may provide a tactile feedback (e.g., false stop) to the operator or generate an audible click to provide audible feedback to the operator. The operator can stop at a given clock hour and dispense a given volume of viscoelastic or other fluid, and then move to the next clock hour.

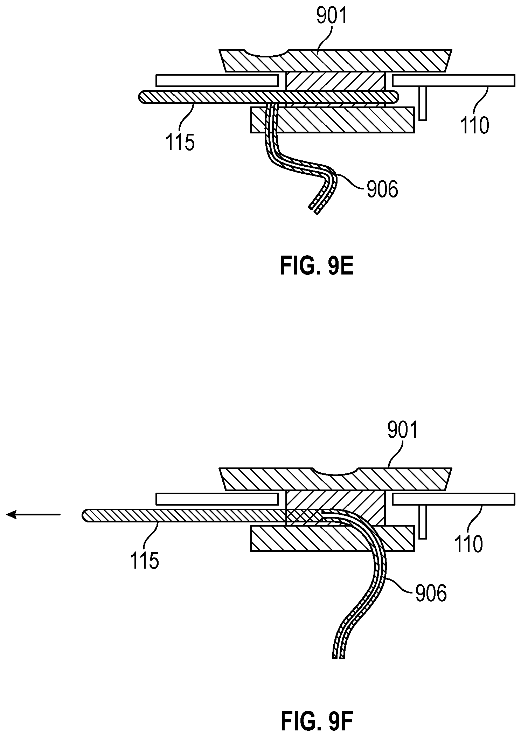

The catheter 115 may be made of one or multiple joined materials, such as nylon/Pebax^{®} polymer, polyimide, etc. The proximal end of the catheter 115 may be overmolded (e.g., insert molded) into or bonded to (e.g., via an adhesive) the dial mechanism. The channeling to feed the catheter 115 into the outer cannula 120 may be a separate molded or metallic component, or may be formed with the housing 110 of the cannula adapter 100. FIGURES 9E and 9F illustrate an example of how the dial 901 may be incorporated into or assembled with the housing 110. The inner catheter 115 may be connected to a fluid delivery system (not shown) such as a valve or syringe (such as those described herein) via a separate section of tubing 906 fluidically coupled to the catheter 115 (as shown in FIGURES 9E and 9F) or via channels formed into the molded components. In some configurations, a valve may be directly integrated into the dial mechanism.

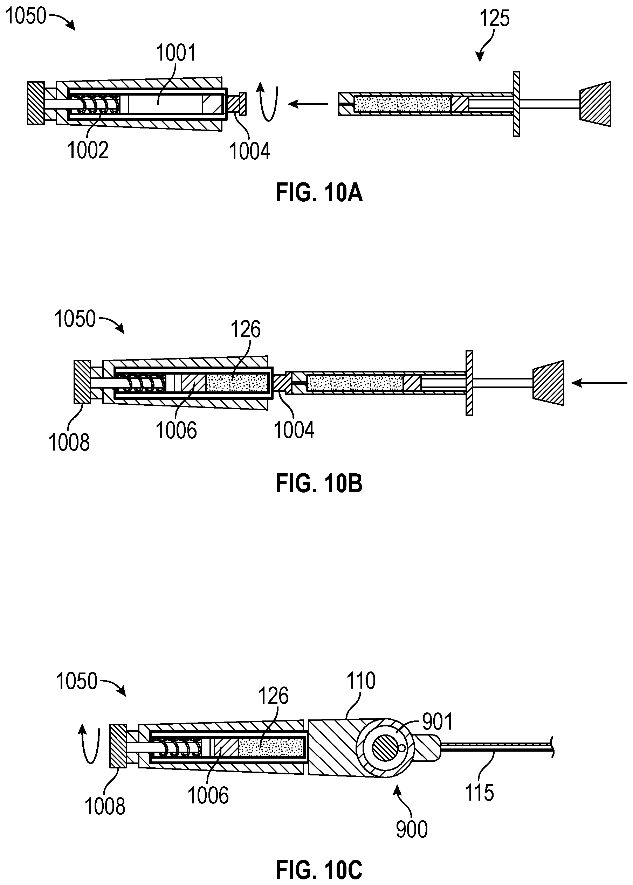

The cannula adapters described and illustrated herein may incorporate a variety of different viscoelastic dispensing mechanisms or systems. **FIGURES 10A-****10C** illustrate an embodiment of a delivery system 1050 in conjunction with the cannula adapter 900 of FIGURES 9A-9F. The delivery system 1050 can includes a reservoir 1001, a spring 1002, a connector 1004, a stopper 1006, and a base member 168. The reservoir 1001 (or cartridge) that may be removably coupled (e.g., friction fit, corresponding mating features, latches and notches, etc.) to the housing 110 of the cannula adapter 900. The reservoir 1001 can include a spring 1002, which can be retracted to a compressed position (or configuration) before use as shown in FIGURE 10A. The stopper 1006 (e.g., rubber stopper) can be positioned within and near the proximal end (e.g., an end opposite from the feed mechanism 900) of the reservoir 1001. The connector 167 (e.g., a female Luer connector) can be placed at the distal end (e.g., an end proximate to the feed mechanism 900) of the reservoir 1001 to facilitate direct connection to any standard viscoelastic syringe or other viscosurgical device (e.g., a pre-filled ophthalmic viscoelastic device 125). In some implementations (not shown), a septum may be present at the distal end of the reservoir 1001 that can be pierced with a needle or dispensing cannula.

The reservoir 1001 may be filled with fluid 126 (e.g., viscoelastic or other ophthalmic viscosurgical device (OVD) fluid) by depressing the plunger of the standard viscoelastic syringe 125. As the reservoir 1001 is filled, the stopper 1006 can be pushed back to the compressed spring 1002. A bleed valve or air breathable material, such as a porous plastic or porous polymer material, may be integrated into the reservoir 1001 to allow further purging of air during the fill. Once a desired amount of the fluid 126 (e.g., viscoelastic or other fluid) is added to the reservoir 1001, the delivery system 1050 can be connected to the housing 110 including the catheter 115. Once the delivery system 1050 is connected to the housing 110, the base member 1008 can be rotated to release the spring 1002 to apply force to the stopper 1006. The spring 1002 may be operatively coupled to the base member 1008 so as to facilitate engagement of (e.g., compression and relaxation) the spring 1008 upon actuation of the base member 168. In some implementations, rotation of the base member 1008 can cause compression or relaxation of the spring 1008. In accordance with several embodiments, the delivery system 1050 of FIGURES 10A-10C can advantageously accommodate any choice of viscoelastic or other fluid and different amounts of viscoelastic or other fluid. The delivery system 1050 can ensure that a consistent spring force is applied to the reservoir 1001 when filled.

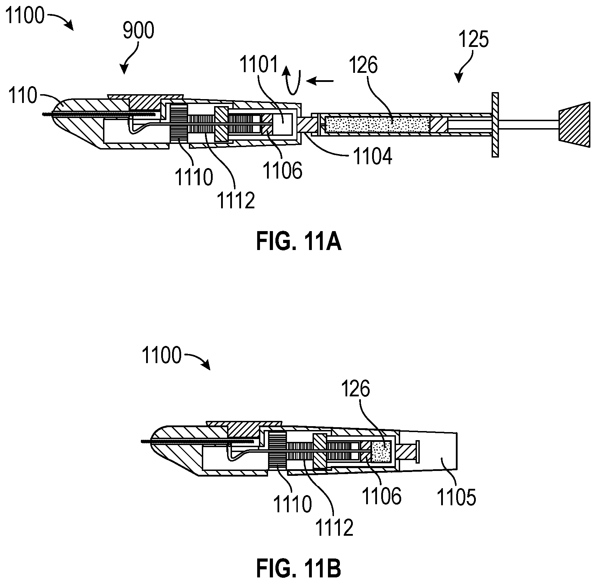

With reference to **FIGURES 11A and 11B****,** a fillable cannula adapter 1100 is disclosed herein. The embodiment of the cannula adapter 1100 illustrated in FIGURES 11A and 11B utilizes a screw-driven (or threaded plunger) system that can be incorporated into the housing 110 of the cannula adapter 1100 or housings of other embodiments of cannula adapters described herein. The cannula adapter 1100 can include a threaded plunger 1112, a wheel 1110, a reservoir 1101, and a connector 1104 (e.g., standard Luer connector or check valve). The reservoir 1101 can be filled (e.g., primed) with the fluid 126 from the syringe 125 (e.g., a standard viscoelastic syringe) and a cap or other sealing member can be placed on a back end of the reservoir 1101 prior to operation. The wheel 1110 may be located and positioned such that the wheel 1110 is accessible via a slot formed in a belly (e.g., bottom side or lower side) of the housing 110. The wheel 1110 may be conveniently positioned as to be rotated by an index finger or middle finger of the operator as the housing 110 is held in the operator's hand with the thumb over the dial 900. The threaded plunger 1112 can be operatively and mechanically coupled to the wheel 1110 such that the threaded plunger 1112 is actuated by rotation of the wheel 1110. The threaded plunger 1112 and the wheel 1110 can include a lumen that is fluidically coupled to tubing (e.g., flexible silicone tubing) that is in turn fluidically coupled to the lumen of the catheter 115. In some implementations, the lumen of the threaded plunger 1112 may be directly coupled to the lumen of the catheter 115 without an intermediate tubing. As the threaded plunger 1112 pushes on the reservoir 1101, the fluid 126 (e.g., viscoelastic or other ophthalmic viscosurgical device (OVD) fluid) is dispensed back through the plunger 1112 toward the catheter 115.

Although the fluid delivery system is illustrated in FIGURES 11A and 11B is coupled to the feed mechanism 900 described and illustrated in connection with FIGURES 9A-9F, the fluid delivery system of FIGURES 11A and 11B could theoretically work with any suitable feed system embodiment. In accordance with several embodiments, the catheter 115 is able to move independent of the threaded plunger 1112 (e.g., via the feed mechanism 900), such that the operator can dispense fluid at any time. This independent movement capability may be performed by linking the plunger tube (e.g., a tube that extends through the lumen of the wheel 1110 and the plunger 1112) to the catheter 115 via flexible tubing (e.g., silicone tubing) with slack. The connection between the plunger tube and the catheter 115 may alternatively be made with Pebax^{®} polymer tubing or other more durable tubing, or with rigid tubing shaped like a spring to allow independent motion of the catheter 115.

The connector 1104 (e.g., a female connector) can allow the operator to fill the reservoir 1101 with any standard viscoelastic syringe 125, as with the delivery system 1050 illustrated in FIGURES 10A-10C. The reservoir 1101 and entire downstream system could be primed with the syringe 125. The reservoir 1101 and the stopper 1106 (e.g., rubber stopper) may be shaped such to streamline the filling procedure and prevent unwanted trapped air. The operator could then detach the viscoelastic syringe 125 and replace with a plug or cap 1105. The plug or cap 1105 may be shaped to form the rest of the handle for ergonomics. The cap 1105 may also have an extension that protrudes into the reservoir 1101 to purge any last bit of air from the connection when attached. Alternatively, the device may include a valve that can be closed when priming is complete.

The wheel 1110 of the housing 110 (e.g., exposed on the belly of the housing 110) may include detents to provide an indication of volume dispensed. For example, the detents can generate audible clicking noises or tactile feedback at each incremental volume. The detents may be user customizable such that the operator can choose to have a click at each 2uL or a click at each 5uL, for example. Of course, other volume indications may be used as desired and/or required. In some embodiments, the belly (or lower surface) of the housing 110 may include multiple spaced-apart linear indicators that show the total volume dispensed thus far or remaining volume left in the reservoir (e.g., the reservoir 1101). This mechanism could be driven by a coupled screw with a coarser pitch to amplify the linear motion, or on a gear and pinion system, for example.

In accordance with several embodiments, the cannula adapter 1100 illustrated in FIGURES 11A and 11B can advantageously include a fluid delivery system configured for single-handed use that should be familiar to the operator. The fluid delivery system also allows dispensing of the viscoelastic or other fluid at any time as opposed to being coupled to the motion of the inner catheter, thereby allowing the operator to move through a barrier or obstruction that the inner catheter hits, or contact, while traversing along Schlemm's canal.

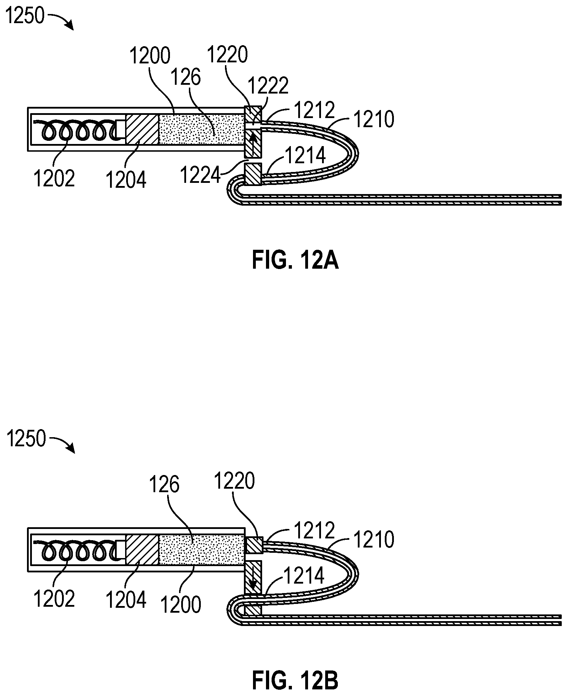

**FIGURES 12A-12B** illustrate an embodiment of a delivery system 1250 that may be incorporated into a cannula adapter described herein. The delivery system 1250 may advantageously allow for controlled microbolus dispensing. The delivery system 1250 can include an elastic (e.g., compliant) tube 1210 that is charged by closing a distal end 1214 of the tube 1210 and opening a proximal end 1212 of the tube 1210 to a pressurized (e.g., spring loaded) reservoir 1200. The reservoir 1200 may include a spring 1202 and a stopper 1204 with structural and operational features similar to the delivery system 1050 described in connection with FIGURES 10A-10C. After charging of the tube 1210 as shown in FIGURE 12A, the operator may toggle a switch (not shown) to close the proximal end 1212 and open the distal end 1214 to discharge the elastic tube 1210 through the catheter 115. The length, diameter, and durometer of the elastic tube 1210 can be varied to achieve the desired bolus volume.

The opening and closing of the proximal end 1212 and the distal end 1214 can be facilitated by causing a controller to move a block 1220 between different positions. For example, the block 1220 can have a first position (as shown in FIGURE 12A) and a second position (as shown in FIGURE 12B). When the block 1220 is in the first position, a first opening 1222 of the block 1220 can fluidically connect the proximal end 1212 of the tube 1210 and the reservoir 1200 while a second opening 1224 can be offset from the distal end 1214 to fluidically disconnect the distal end 1212 and the catheter 115. As such, when the block 1220 is in the first position, the fluid 126 (e.g., viscoelastic or other ophthalmic viscosurgical device (OVD) fluid) stored in the reservoir 1200 can enter into the tube 1210 via the proximal end 1212 and fill the tube 1210 up to the distal end 1214. When the block 1220 is in the second position, the first opening 1222 can be offset from the proximal end 1212 to fluidically disconnect the reservoir 1200 and the proximal end 1212 while the second opening 1224 can fluidically connect the distal end 1214 and the catheter 115. As such, when the block is in the second position, fluid that was stored within the tube 1200 (e.g., between the proximal end 1212 and the distal end 1214) can be discharged into the catheter 115 via the second opening 1224.

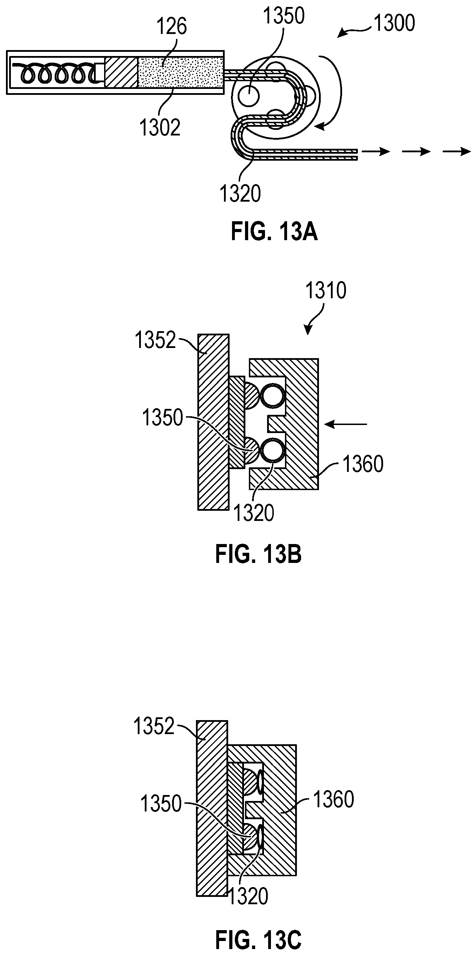

**FIGURES 13A-13C** illustrate an embodiment of a peristaltic fluid delivery system 1300. The peristaltic fluid delivery system 1300 can include an elastic (or compliant) tube 1320 and a peristaltic pump 1310 (shown in FIGURES 13B and 13C) that includes protrusions 1350 (e.g., rollers or ball bearings) adapted to sequentially compress or squeeze the tube 1320 at spaced-apart locations along a length of the tube 1320 so as to convey liquid between a fluid reservoir 1300 storing the fluid 126 and the catheter 115. At least a portion of the tube 1320 and the protrusions 1350 may be encased within a circular pump casing 1360. The peristaltic pump 1310 may be a rotary peristaltic pump (as shown) or a linear peristaltic pump.

In the illustrated embodiment, the tube 1320 can be positioned within the pump casing 1360 about the protrusions 1350 (e.g., ball bearings) on a rotor 1352. The pump casing 1360 can be actuated (e.g., pushed towards the rotor 1352) to pinch (e.g., compress) at least a portion of the tube 1320 with the protrusions 1350. While the pump casing 1360 is actuated, rotation of the rotor 1352 force the fluid 126 downstream along the tube 1320. As each of the pump elements 1350 is disengaged with the tube 1320, fluid flow is induced into that respective portion of the tube 1320. FIGURES 13B and 13C illustrate cross-section views of the peristaltic pump 1310. The peristaltic fluid delivery system 1300 may be primed (e.g., allow the fluid 126 to enter into the tube 1320) prior to the tube 1320 engaging the pump elements 1350. In some embodiments, the pump 1310 can be actuated by rotating the rotor 1352 after priming.

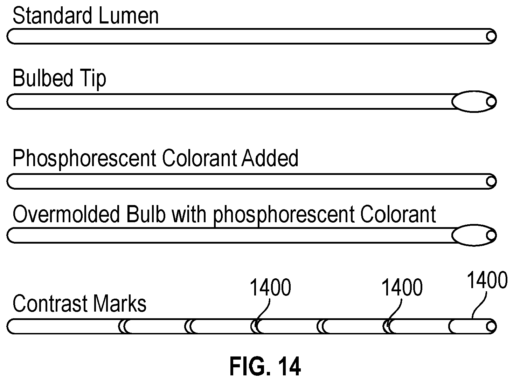

**FIGURE 14** illustrates various concepts related to catheter shape and features to facilitate visualization or tracking of the catheter 115 as it is advanced along Schlemm's canal. A key challenge of a visco delivery or other fluid delivery procedure is knowing where the tip of the inner catheter is so that it doesn't migrate to undesirable anatomy (such as the suprachoroidal space when the target is Schlemm's canal). The top image shows a standard catheter lumen. The second image from the top shows that a distal end of the inner catheter may include a bulbed tip. The bulbed tip may be achieved by overmolding material onto an extruded lumen. With reference to the third image in the middle, the catheter itself (e.g., the catheter 115) may be formed using a polymer with a phosphorescent colorant such that it can be charged with a microscope light or an ultraviolet light/blue light source and then can glow when the lights are dimmed. The operator may alternatively have a UV/blue light source attached to a surgical microscope that can be switched on during a procedure to better view the phosphorescent catheter. In the embodiment illustrated in the fourth image (second from the bottom), the catheter includes a bulbed distal tip that is colored with the phosphorescent colorant (e.g., glow-in-the-dark material such as strontium aluminate). The bulbed tip may be overmolded on a distal end of an extruded lumen. The final image on the bottom shows that the catheter 115 may include contrast marks 1400 spaced apart along a length of the inner catheter to facilitate visualization of the inner catheter (e.g., more clearly denote which part of the inner catheter you are seeing through the trabecular meshwork). The distal tip may include a more extensive (e.g., longer or wider) contrast mark to indicate the distal tip of the inner catheter. The cannula adapters described herein may be designed for use without a fiber optic cable so as to reduce cost.

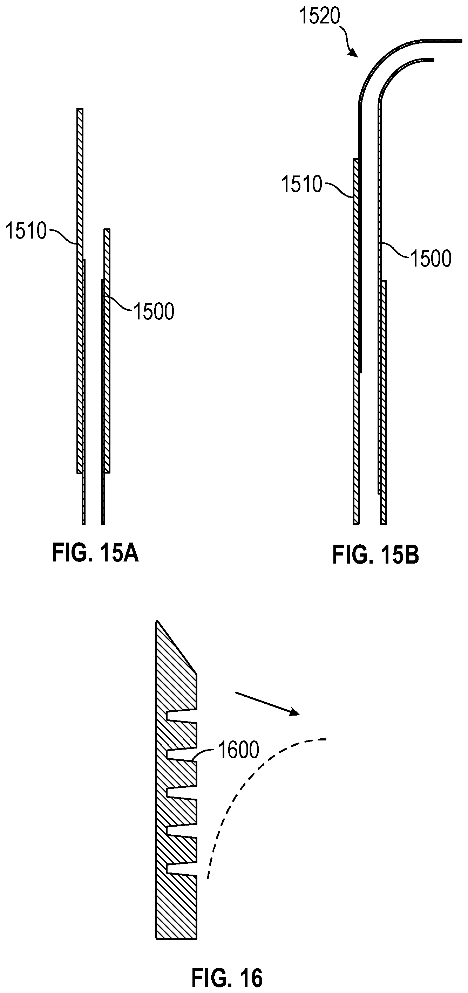

**FIGURES 15A and 15B** schematically illustrate operation of an outer cannula 1500 that utilizes shape memory material (e.g., nitinol or other shape memory alloy material) to form a shape set distal tip 1520. The distal tip 1520 of the cannula can be straight when confined by an introducer needle 1510 (e.g., 304 Stainless steel material, other 300 Series Stainless steel material or other material) but curves to a set shape when no longer constrained (e.g., by the introducer needle). This shape set configuration allows the outer cannula 1500 to slide within a rigid introducer needle 1510, but curve to enter Schlemm's canal when slid forward out of the introducer needle 1510.

**FIGURE 16** illustrates an embodiment of a distal end portion (e.g., distal tip) of the outer cannula 120. As shown, the distal end portion (e.g., distal tip) may include notches 1600 in the tubing to allow the cannula 120 to articulate in a particular direction. In some implementations, the distal end portion (e.g., distal tip) is connected to a pull wire such that the operator can control articulation of the cannula 120 near the notched distal tip. In some implementations, the tip of the outer cannula 120 may be configured to seat or anchor to the back wall of Schlemm's canal to prevent inadvertent motion of the tip during, for example, a visco or other fluid delivery procedure.

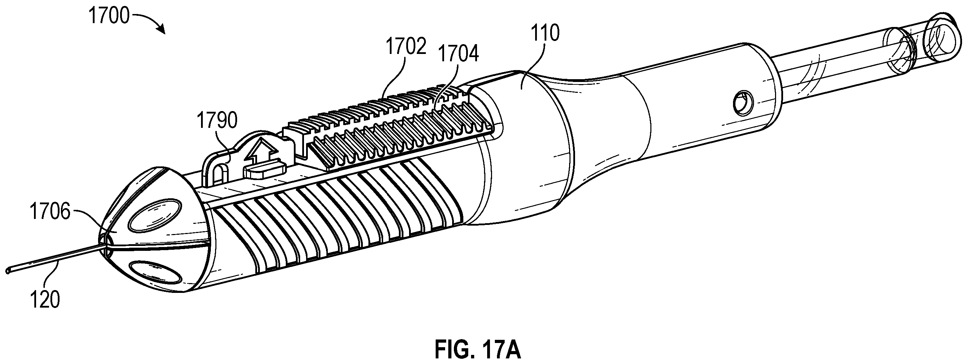

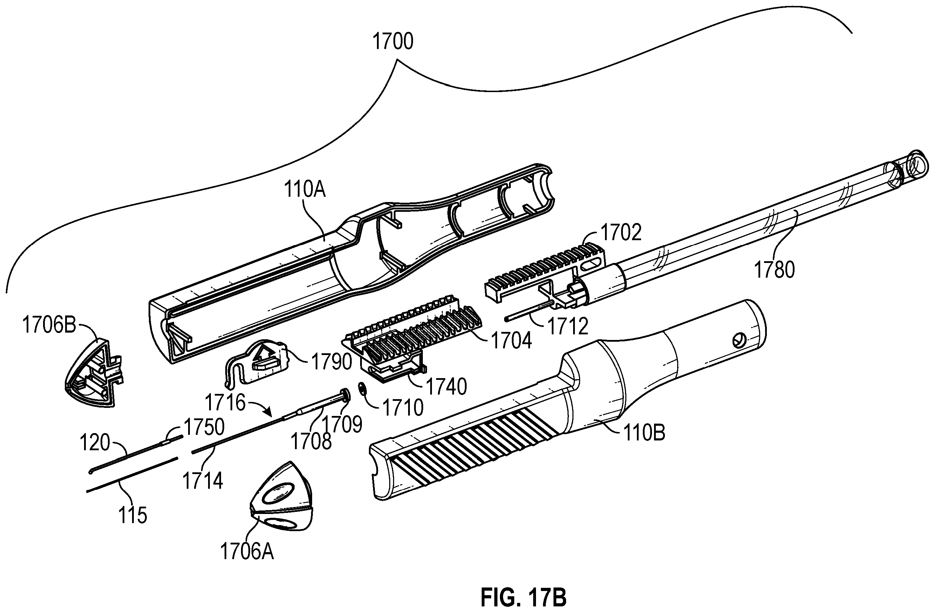

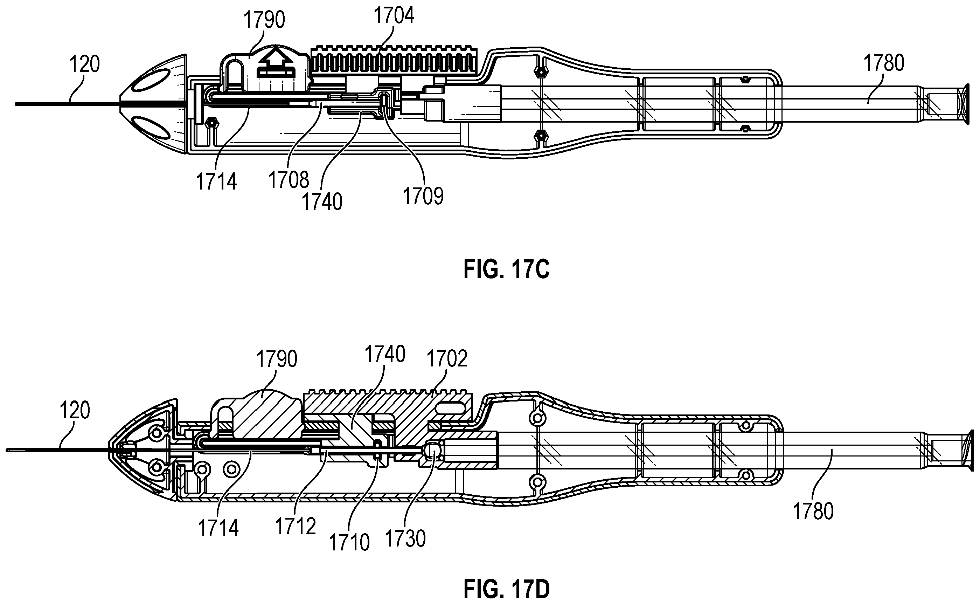

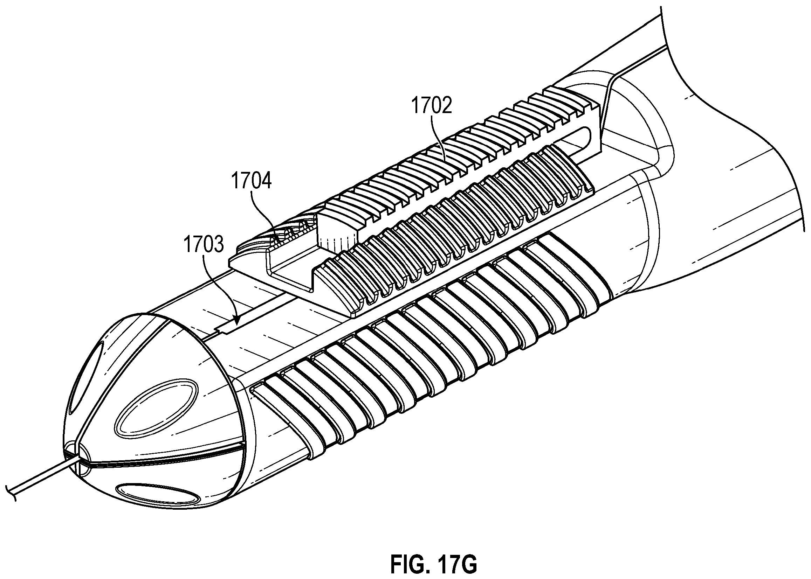

With reference to **FIGURES 17A-17H****,** an embodiment of a cannula adapter, or fluid delivery device, 1700 is disclosed. The cannula adapter 1700 can include a housing 110, a first slider 1702, a second slider 1704, a channel 1703, a fluid delivery conduit 1716, a stopper 1790, and a distal end 1706. The distal end 1706 can include an opening sized to receive a cannula 120. The first slider 1702 can be mechanically and/or operably coupled to a reservoir 1780 and a plunger 1712. The second slider 1704 can include an insert 1740 that can be formed on, for example, an underside of the second slider 1704.

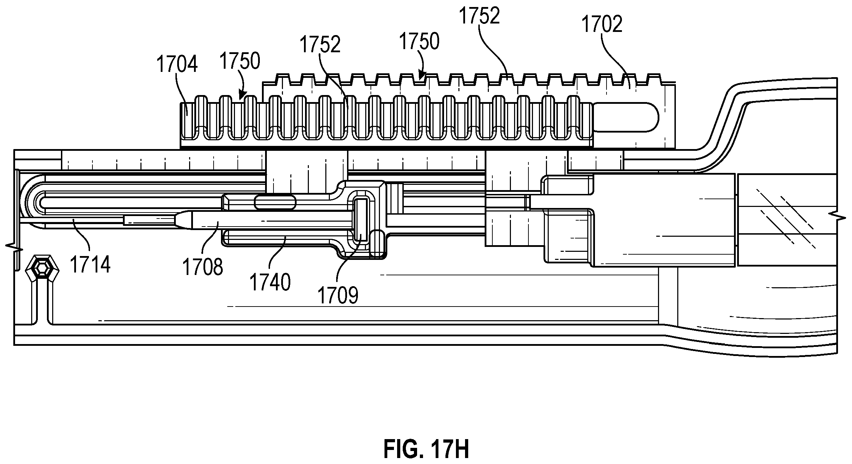

The fluid delivery conduit 1716 can include a proximal base 1709, a body 1708, and a tube 1714. The fluid delivery conduit 1716 can be positioned within the insert 1740 of the second slider 1704 (as shown in FIGURE 17H). In some implementations, at least a portion of the body 1708 and the base 1709 is positioned within the insert 1740 of the second slider 1704. The tube 1714 of the fluid delivery conduit 1716 can be operably coupled with the catheter 115 such that distal and proximal movement of the fluid delivery conduit 1716 can distally or proximally move the catheter 115. In some implementations, the tube 1714 is directly connected to the catheter 115. In some implementations, the tube 1714 is connected indirectly to the catheter 115 via an intervening tube. The tube 1714 is fluidly coupled to the catheter 115.

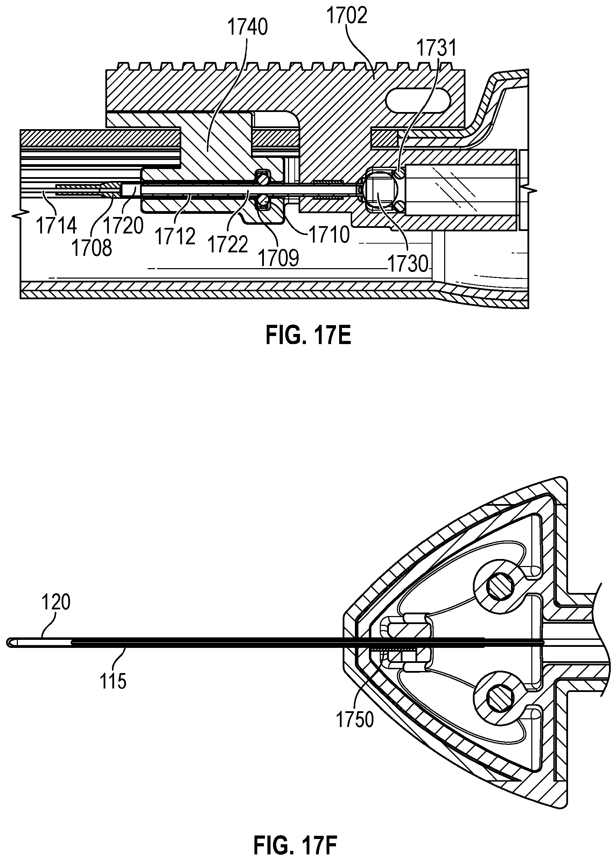

The fluid delivery conduit 1716 can allow fluid (e.g., viscoelastic fluid) to flow from the reservoir 1780 to the catheter 115. As shown in FIGURES 17D and 17E, the body 1708 of the fluid delivery conduit 1716 can include a bore 1720 that can receive the plunger 1712 that can slide within the bore 1720 of the body 1708. The plunger 1712 can include a lumen 1722 that can allow fluid (e.g., viscoelastic fluid) to flow through the plunger 1712, into the bore 1720 of the fluid delivery conduit 1716, and through the tube 1714 towards the catheter 115.

During operation, the first slider 1702 and the second slider 1704 can together move distally (e.g., towards the distal end 1706) along the channel 1703. As described herein, the distal movement of the second slider 1704 can cause distal movement (e.g., moving towards the distal end 1706) of the fluid delivery conduit 1716, which can in turn cause the catheter 115 to move distally and out of the cannula 120. With reference to the example illustrated in FIGURE 1F, an operator can penetrate a superior trabecular meshwork (TM) portion with the distal portion 121 of the cannula 120 and slide the first slider 1702 and the second slider 1704 to guide the catheter 115 out of the cannula 120 and into the Schlemm's canal (SC).

Once the catheter 115 is extended out of the cannula 120 (e.g., guided into the Schlemm's canal (SC)), the first slider 1702 can be used to dispense fluid (e.g., viscoelastic fluid) stored in the reservoir 1780. To dispense fluid stored in the reservoir 1780, the first slider 1702 may be moved proximally along the channel 1703 (e.g., away from the distal end 1706) and subsequently moved distally (e.g., towards the distal end 1706) along the channel 1703 relative to the second slider 1704. In some implementations, the second slider 1704 remains stationary (e.g., to ensure that the catheter 115 does not move) while the first slider 1702 is moved along the channel 1703 to dispense fluid (e.g., the fluid 126) stored in the reservoir 1780. When the first slider 1702 is moved proximally along the channel 1703 relative to the second slider 1704, the plunger 1712 can move proximally within the bore 1720 of the body 1708 of the fluid delivery conduit 1716. The proximal movement of the plunger 1712 (e.g., relative to the fluid delivery conduit 1716) within the bore 1720 can cause the fluid stored in the reservoir 1780 to flow past a ball 1730 and a gasket 1731, into the lumen 1722 of the plunger 1712, and into the bore 1720 of the fluid delivery conduit 1716. Once the fluid flows into the bore 1720 of the fluid delivery conduit 1716, the plunger 1712 can moved distally along the channel 1703 to force the fluid out from the bore 1720 and into the tube 1714. In some implementations, the ball 1730 (and the gasket 1731) can limit the amount of fluid flowing from the reservoir 1780 and into the lumen 1722 of the plunger 1712.

In some implementations, an operator (e.g., a clinician, surgeon, care provider) can change the amount of dispensed fluid by changing the distance travelled by the first slider 1702 along the channel 1703. For example, the further the first slider 1702 is moved proximally (e.g., towards the operator) along the channel 1703, the more fluid is dispensed via the catheter 115, and vice versa. In some implementations, the channel 1703 (or the first slider 1702) can include detents that can provide tactile feedback as to how far the first slider 1702 is moved proximally (e.g., towards the operator) along the channel 1703 to indicate how much fluid will be dispensed once the first slider 1702 is moved back distally along the channel 1703.

The ball 1730 and the gasket 1731 can together function as a check valve for the plunger 1712. The ball 1730 and the gasket 1731 can allow the fluid (e.g., the fluid 126) to be dispensed from the reservoir 1780 while preventing fluid (e.g., the fluid 126) from flowing backwards (e.g., towards the reservoir 1780) past the ball 1730 and the gasket 1731. As such once the fluid flows past the ball 1730 and the gasket 1731, it cannot flow back into the reservoir 1780. In some implementations, other types of suitable valves may be used to provide unidirectional flow of the fluid (e.g., the fluid 126) out of the reservoir 1780 and through the plunger 1712.

In some implementations, a user may experience increased resistance when sliding the second slider 1704 back and forth to dispense the fluid from the reservoir 1780 than when sliding both the first slider 1702 to move the catheter 115 out of the cannula 120. The resistance may be caused by greater friction between the second slider 1704 and the first slider 1702 (and the channel 1703) than between the first slider 1702 and the channel 1703. This difference in resistance can provide, for example, tactile feedback that can allow a user to distinguish the catheter dispensing motion (e.g., sliding the first slider 1702) and the fluid dispensing motion (e.g., sliding the second slider 1704).

In some implementations, the second slider 1704 can include a groove 1705 through which the first slider 1702 can move (e.g., slide) distally or proximally. The first slider 1702 and the second slider 1704 can include ridges 1752 and grooves 1750 that can provide better grip for an operator (e.g., care provider).

In some implementations, the cannula 120 can include a protrusion 1750 that can fixedly attach the cannula 120 to an insert formed inside the distal end 1706 (as shown in an example illustrated in FIGURE 17F). As such, the distal end 1706 can be rotated to change the orientation of the cannula 120 and therefore the orientation of the distal portion 121 of the cannula 120. This can advantageously allow an operator to change the orientation of the cannula 120 by rotating the distal end 1706 without having to change the orientation (e.g., rotating about an axis parallel with the length of the cannula adapter) of the cannula adapter 1700.

The cannula adapter 1700 can include a stopper 1790 that can be placed inside the channel 1703. The stopper 1790 can prevent distal movement of the first slider 1702 and the second slider 1704 during, for example, storage or operation. This can advantageously prevent the catheter 115 from accidentally or inadvertently extending out from the cannula 120.

As shown in an example in FIGURE 17E, a gasket 1710 may be placed inside the base 1709 of the fluid delivery conduit 1716. The gasket 1710 may be dimensioned to fit snugly within the base 1709 and include an opening (e.g., circular opening) to receive the plunger 1712. In some implementations, the gasket 1710 can contact the outer surface of the plunger 1712 to generate sufficient resistance to cause smooth, controlled movement of the plunger 1712 within the bore 1720 of the body 1708.

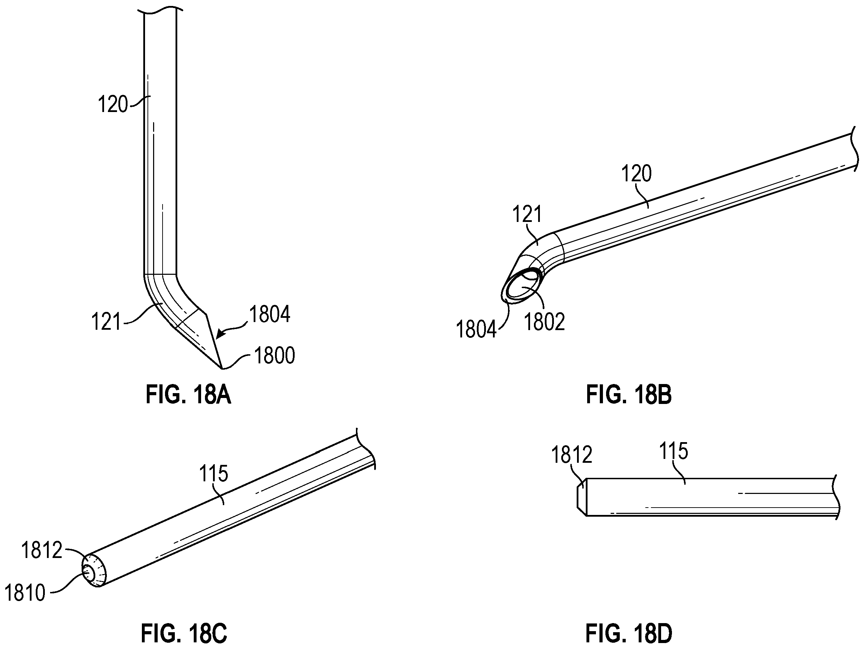

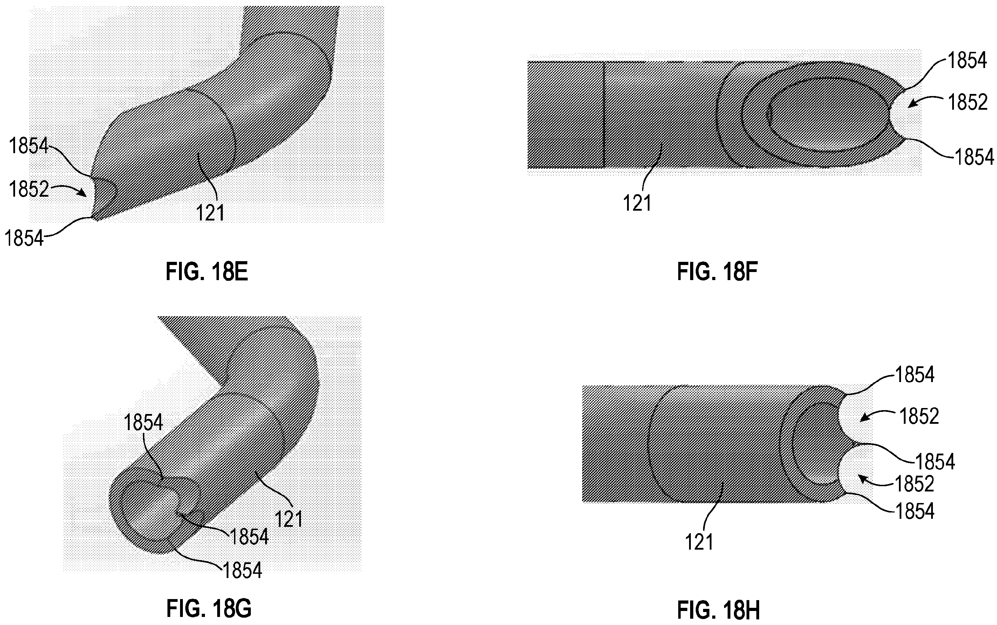

**FIGURES 18A-18D** illustrate various views of the cannula 120 and the catheter 115. As described herein, the cannula 120 can include the distal portion 121 that can penetrate a superior trabecular meshwork (TM) portion with a tip 1800 and an end surface 1804. The end surface 1804 may be formed at an angle (e.g., slanted) with respect to an axis parallel to the body of the cannula 120. The surface 1804 can facilitate, for example, penetration of a superior trabecular meshwork (TM) portion. In some implementations, the end surface 1804 may be orthogonal (or substantially orthogonal) with respect to an axis parallel to the body of the cannula 120. The tip 1800 may form a beveled distal tip to facilitate a cutting edge. The distal portion 121 may be curved to allow the catheter 115 to extend out (e.g., exit) from the lumen 1802 of the cannula 120 at an angle, which can facilitate movement of the catheter 115 into and around the Schlemm's canal (SC). The catheter 115 can include a distal end 1810 having a tapered edge 1812. The tapered edge 1812 can facilitate and guide movement of the catheter 115 within the curved distal portion 121 and inside the Schlemm's canal (SC).

**FIGURES 18E-18H** illustrate other examples of the distal portion 121 of the cannula 120. The distal portion 121 can include one or more cutouts 1852 that can be formed at the tip 1800 of the distal portion 121 to provide one or more cutting edges or surfaces. In some implementations, the cutouts 1852 can be formed on a distal edge (e.g., the edge further from a portion of the cannula 120 proximate to the distal portion 121) of the distal portion 121. In some implementations involving multiple cutouts 1852, the cutouts 1852 can be formed adjacent to one another (e.g., as shown in FIGURES 18G and 18H). The cutouts 1852 can include anchors 1854 that can abut against a surface (e.g., the back wall of Schlemm's canal) and stabilize the cannula 120 (e.g., prevent the distal portion 121 of the cannula 120 from moving around) during dispensing of the fluid via a catheter (e.g. the catheter 115). In some implementations, the anchors 1854 can, during use, penetrate a tissue surface (e.g., the back wall of Schlemm's canal). The distal portions 121 described herein and shown in FIGURES 18E-18H may be incorporated in any implementations of the cannula 120 described herein.

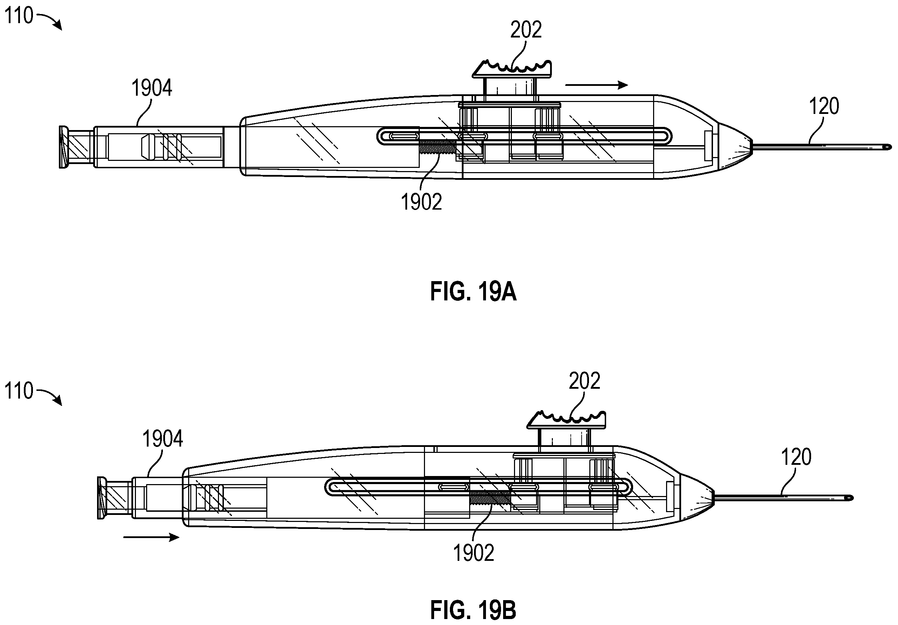

**FIGURES 19A and 19B** illustrate an example operation scheme of a cannula adapter 1900. The cannula adapter 1900 can include the slider 202, the cannula 120, a dispensing mechanism 1902 and a reservoir 1904. An operator can slide the slider 202 distally (e.g., towards the patient) to guide, for example, the catheter out of the cannula 120 and into the Schlemm's canal. Subsequently, the operator can actuate the reservoir 1904 to dispense fluid into and out of the catheter 115. In some implementations, the dispensing mechanism 1902 may include a fixed plunger positioned within the reservoir 1904 that pushes the fluid out of the reservoir 1904 when the reservoir 1904 rotated about an axis parallel to the length of the cannula adapter 1900. In some implementations, the dispensing mechanism 1902 may include a plunger positioned within the reservoir 1904 and connected to an elastic member (e.g., a spring) that can push the fluid out of the reservoir 1904 and towards the catheter 115 when, for example, a proximal end of the reservoir 1904 is pushed distally (e.g., towards the patient).

In accordance with several implementations, Trypan blue or some other biocompatible dye (e.g., brilliant blue, indocyanine green, fluorescein) into the reservoir 164, prior to filling. Introducing biocompatible dye may allow the operator to visualize the extent of dilation in Schlemm's canal and the downstream episcleral venous network.

Several additional features may be integrated with the above concepts. For example, wound sealing features outside of the outer cannula 120 or introducer needle, such as a compliant/elastomer overmold or o-ring that fits within or presses against the corneal incision during surgery to prevent aqueous humor leakage. Alternatively, a balanced salt solution (BSS) infusion path through the outer cannula 120 may be used to provide chamber stability.

Although primarily described with respect to delivery of viscoelastic within Schlemm's canal, the devices and methods described and illustrated herein could be used in connection with delivery of viscoelastic or other fluid to other pre-existing or created anatomical passages, channels, spaces, lumens, or vessels either associated with the eye (e.g., collector channels, downstream episcleral venous networks, tissue tracts of the eye, suprachoroidal space, subconjunctival space, subretinal space) or in other locations other than the eye. Fluids other than viscoelastic may be used (e.g., other liquid drug, medicament, solution, chemical, etc.).

Conditional language such as, among others, "can," "could," "might" or "may," unless specifically stated otherwise, are otherwise understood within the context as used in general to convey that certain embodiments include, while other embodiments do not include, certain features, elements and/or steps. Thus, such conditional language is not generally intended to imply that features, elements and/or steps are in any way required for one or more embodiments or that one or more embodiments necessarily include logic for deciding, with or without user input or prompting, whether these features, elements and/or steps are included or are to be performed in any particular embodiment.

Conjunctive language such as the phrase "at least one of X, Y and Z," unless specifically stated otherwise, is otherwise understood with the context as used in general to convey that an item, term, etc. may be either X, Y or Z. Thus, such conjunctive language is not generally intended to imply that certain embodiments require at least one of X, at least one of Y and at least one of Z to each be present.

Some embodiments have been described in connection with the accompanying drawings. However, the figures are not drawn to scale. Distances, angles, etc. are merely illustrative and do not necessarily bear an exact relationship to actual dimensions and layout of the devices illustrated. Components can be added, removed, and/or rearranged. Further, the disclosure herein of any particular feature, aspect, method, property, characteristic, quality, attribute, element, or the like in connection with various embodiments can be used in all other embodiments set forth herein.

## Claims

1. A device (100) for delivering fluid, the device comprising:
a housing (110);
a fillable reservoir (1001) for storing fluid and a connector (1004) configured to be fluidically coupled to a pre-filled ophthalmic viscoelastic device (125);
a cannula (120) comprising a proximal end, a distal end, and a lumen, the proximal end of the cannula (120) coupled to a distal end of the housing (110), the distal end of the cannula (120) configured to penetrate an eye of a patient via a corneal incision, and the lumen of the cannula (120) extending from the proximal end to the distal end of the cannula (120);
a catheter (115) comprising a proximal end, a distal end, and a lumen, the proximal end of the catheter (115) fluidically coupled to the reservoir (1001), and the lumen extending from the proximal end of the catheter (115) to the distal end of the catheter (115);
a first actuator (105) configured to, when actuated, advance the catheter (115) along the lumen of the cannula (120) and cause the catheter (115) to exit via the distal end of the cannula (120); and
a second actuator (128) configured to, when actuated, cause the fluid stored in the reservoir (1001) to flow through the catheter (115) and exit via the distal end of the catheter (115),
wherein the lumen of the catheter (115) is configured to allow the fluid stored in the reservoir (1001) to flow through the catheter (115) and into the eye of the patient,
wherein the catheter (115) is sized so as to extend out of the distal end of the cannula (120) and along an entire 360-degree circumference of Schlemm's canal of the patient when the first actuator (105) is actuated,
wherein the first actuator (105) and the second actuator (128) are configured to be actuatable by a single hand of the user, and
wherein the first actuator (105) can be moved relative to the second actuator (128), such that dispensing of the fluid stored in the reservoir (1001) is decoupled from catheter (115) motion.

2. The device (100) of Claim 1, wherein the distal end of the cannula (120) comprises a scoop with a sharpened tip.

3. The device (100) of any of Claims 1-2, wherein the distal end of the cannula (120) is pre-curved.

4. The device (100) of any of Claims 1-3, wherein at least a distal portion of the catheter (115) is pre-shaped to follow approximately a radius of curvature of Schlemm's canal.

5. The device (100) of any of Claims 1-4, wherein at least a distal portion of the catheter (115) is flexible.

6. The device (100) of any of Claims 1-5, wherein the cannula (120) comprises rigid material.

7. The device (100) of any of Claims 1-6, wherein the distal end of the cannula (120) comprises a cutout and two or more anchors.

8. The device (100) of any of Claims 1-7, wherein:
the housing (110) further comprises a channel;
the first actuator (105) comprises a first sliding trigger adapted to translate axially within the channel between a proximal position and a distal position; and
the second actuator (128) comprises a second sliding trigger adapted to translate axially within the channel between a proximal position and a distal position.

9. The device (100) of Claim 8, wherein:
when the first sliding trigger is in the proximal position, the distal end of the catheter (115) is positioned between the proximal end and the distal end of the cannula (120);
when the first sliding trigger is in the distal position, the distal end of the catheter (115) is advanced beyond the distal end of the cannula (120);
movement of the second sliding trigger between the proximal position and the distal position causes a predetermined amount of the fluid to be dispensed via the distal end of the catheter (115).

10. The device (100) of Claim 9, wherein an amount of axial movement of the second sliding trigger along the channel corresponds to amount of the fluid dispensed from the catheter (115).

11. The device (100) of Claim 9 or 10, wherein the catheter (115) is positioned and sized such that the distal end of the catheter (115) is adapted to be aligned with the distal end of the cannula (120) when the first actuator (105) is retracted to a proximal-most position within the sliding channel.

12. The device (100) of any of Claims 1 - 11, further comprising a ball and gasket system within the connector (1004) configured to be fluidically coupled to the pre-filled ophthalmic viscoelastic device (125),
wherein the ball and gasket system can allow fluid to flow from the pre-filled ophthalmic viscoelastic device (125) through the distal end of the catheter (115) upon actuation of the second actuator (128), and
wherein the ball and gasket system can prevent fluid to flow from distal end of the catheter (115) to the pre-filled ophthalmic viscoelastic device (125).

13. The device (100) of any of Claims 1- 12, wherein the cannula (120) additionally comprises a cannula protrusion along its distal end;
wherein the housing (110) additionally comprises a proximal portion and a distal portion;
wherein the distal portion of the housing (110) can rotate along an axis parallel with a length of the cannula (120) relative to the proximal portion of the housing (110);
wherein the distal portion of the housing (110) is connected to the cannula (120) protrusion on the cannula (120) such that rotation of the distal portion of the housing (110) changes an orientation of the cannula (120) and therefore an orientation of the distal portion of the cannula (120).

14. The device (100) of any of Claims 1- 13, wherein movement of the second actuator (128) relative to the first actuator (105) is resisted by a differing amount than movement of the first actuator (105) relative to the housing (110).

## Patentansprüche

1. Vorrichtung (100) zur Abgabe von Flüssigkeit, wobei die Vorrichtung umfasst:
ein Gehäuse (110);
ein befüllbares Reservoir (1001) zum Speichern von Flüssigkeit und ein Anschlussstück (1004), das dazu ausgebildet ist, fluidisch mit einem vorgefüllten ophthalmischen viskoelastischen Gerät (125) gekoppelt zu werden;
eine Kanüle (120) mit einem proximalen Ende, einem distalen Ende und einem Lumen, wobei das proximale Ende der Kanüle (120) mit einem distalen Ende des Gehäuses (110) gekoppelt ist, wobei das distale Ende der Kanüle (120) dazu ausgebildet ist, über eine Hornhautinzision in ein Auge eines Patienten einzudringen, und wobei sich das Lumen der Kanüle (120) von dem proximalen Ende bis zu dem distalen Ende der Kanüle (120) erstreckt;
einen Katheter (115) mit einem proximalen Ende, einem distalen Ende und einem Lumen, wobei das proximale Ende des Katheters (115) fluidisch mit dem Reservoir (1001) gekoppelt ist und sich das Lumen von dem proximalen Ende des Katheters (115) bis zu dem distalen Ende des Katheters (115) erstreckt;
ein erstes Betätigungselement (105), das dazu ausgebildet ist, bei Betätigung den Katheter (115) entlang des Lumens der Kanüle (120) vorzuschieben und zu veranlassen, dass der Katheter (115) über das distale Ende der Kanüle (120) austritt; und
ein zweites Betätigungselement (128), das dazu ausgebildet ist, bei Betätigung zu veranlassen, dass die im Reservoir (1001) gespeicherte Flüssigkeit durch den Katheter (115) hindurchfließt und über das distale Ende des Katheters (115) austritt,
wobei das Lumen des Katheters (115) dazu ausgebildet ist, zu ermöglichen, dass die im Reservoir (1001) gespeicherte Flüssigkeit durch den Katheter (115) und in das Auge des Patienten fließt,
wobei der Katheter (115) so dimensioniert ist, dass er sich bei Betätigung des ersten Betätigungselements (105) aus dem distalen Ende der Kanüle (120) heraus und entlang eines gesamten 360-Grad-Umfangs des Schlemm-Kanals des Patienten erstreckt,
wobei das erste Betätigungselement (105) und das zweite Betätigungselement (128) so ausgebildet sind, dass sie mit einer einzigen Hand des Benutzers betätigbar sind, und
wobei das erste Betätigungselement (105) relativ zu dem zweiten Betätigungselement (128) bewegbar ist, sodass die Abgabe der im Reservoir (1001) gespeicherten Flüssigkeit von der Bewegung des Katheters (115) entkoppelt ist.

2. Vorrichtung (100) nach Anspruch 1, wobei das distale Ende der Kanüle (120) einen Löffel mit einer geschärften Spitze umfasst.

3. Vorrichtung (100) nach einem der Ansprüche 1-2, wobei das distale Ende der Kanüle (120) vorgekrümmt ist.

4. Vorrichtung (100) nach einem der Ansprüche 1-3, wobei wenigstens ein distaler Abschnitt des Katheters (115) vorgeformt ist, um ungefähr einem Krümmungsradius des Schlemm-Kanals zu folgen.

5. Vorrichtung (100) nach einem der Ansprüche 1-4, wobei wenigstens ein distaler Abschnitt des Katheters (115) flexibel ist.

6. Vorrichtung (100) nach einem der Ansprüche 1-5, wobei die Kanüle (120) starres Material umfasst.

7. Vorrichtung (100) nach einem der Ansprüche 1-6, wobei das distale Ende der Kanüle (120) einen Ausschnitt und zwei oder mehr Anker umfasst.

8. Vorrichtung (100) nach einem der Ansprüche 1-7, wobei:
das Gehäuse (110) ferner einen Kanal umfasst;
das erste Betätigungselement (105) einen ersten Schiebeauslöser umfasst, der dazu angepasst ist, sich axial innerhalb des Kanals zwischen einer proximalen Position und einer distalen Position zu bewegen; und
das zweite Betätigungselement (128) einen zweiten Schiebeauslöser umfasst, der dazu angepasst ist, sich axial innerhalb des Kanals zwischen einer proximalen Position und einer distalen Position zu bewegen.

9. Vorrichtung (100) nach Anspruch 8, wobei:
wenn sich der erste Schiebeauslöser in der proximalen Position befindet, ist das distale Ende des Katheters (115) zwischen dem proximalen Ende und dem distalen Ende der Kanüle (120) positioniert;
wenn sich der erste Schiebeauslöser in der distalen Position befindet, ist das distale Ende des Katheters (115) über das distale Ende der Kanüle (120) hinaus vorgeschoben;
eine Bewegung des zweiten Schiebeauslösers zwischen der proximalen Position und der distalen Position veranlasst, dass eine vorbestimmte Menge der Flüssigkeit über das distale Ende des Katheters (115) abgegeben wird.

10. Vorrichtung (100) nach Anspruch 9, wobei eine Menge einer axialen Bewegung des zweiten Schiebeauslösers entlang des Kanals einer Menge des aus dem Katheter (115) abgegebenen Flüssigkeit entspricht.

11. Vorrichtung (100) nach Anspruch 9 oder 10, wobei der Katheter (115) derart positioniert und dimensioniert ist, dass das distale Ende des Katheters (115) dazu angepasst ist, mit dem distalen Ende der Kanüle (120) zu fluchten, wenn das erste Betätigungselement (105) in eine proximalste Position innerhalb des Schiebekanals zurückgezogen ist.

12. Vorrichtung (100) nach einem der Ansprüche 1-11, die ferner ein Kugel- und Dichtungssystem innerhalb des Anschlussstücks (1004) umfasst, das dazu ausgebildet ist, fluidisch mit dem vorgefüllten ophthalmischen viskoelastischen Gerät (125) gekoppelt zu werden,
wobei das Kugel- und Dichtungssystem es ermöglichen kann, dass bei Betätigung des zweiten Betätigungselements (128) Flüssigkeit von dem vorgefüllten ophthalmischen viskoelastischen Gerät (125) durch das distale Ende des Katheters (115) fließt, und
wobei das Kugel- und Dichtungssystem verhindern kann, dass Flüssigkeit vom distalen Ende des Katheters (115) zu dem vorgefüllten ophthalmischen viskoelastischen Gerät (125) fließt.

13. Vorrichtung (100) nach einem der Ansprüche 1-12, wobei die Kanüle (120) zusätzlich entlang ihres distalen Endes einen Vorsprung der Kanüle umfasst;
wobei das Gehäuse (110) zusätzlich einen proximalen Abschnitt und einen distalen Abschnitt umfasst;
wobei der distale Abschnitt des Gehäuses (110) relativ zu dem proximalen Abschnitt des Gehäuses (110) um eine zur Längsrichtung der Kanüle (120) parallele Achse drehbar ist;
wobei der distale Abschnitt des Gehäuses (110) mit dem Vorsprung der Kanüle (120) an der Kanüle (120) verbunden ist, sodass eine Drehung des distalen Abschnitts des Gehäuses (110) eine Orientierung der Kanüle (120) und damit eine Orientierung des distalen Abschnitts der Kanüle (120) ändert.

14. Vorrichtung (100) nach einem der Ansprüche 1-13, wobei einer Bewegung des zweiten Betätigungselements (128) relativ zu dem ersten Betätigungselement (105) ein anderer Widerstand entgegengesetzt ist als einer Bewegung des ersten Betätigungselements (105) relativ zu dem Gehäuse (110).

## Revendications

1. Dispositif (100) pour administrer un fluide, le dispositif comprenant :
un boîtier (110) ;
un réservoir (1001) remplissable pour stocker un fluide et un connecteur (1004) configuré pour être couplé fluidiquement à un dispositif viscoélastique ophtalmique (125) prérempli ;
une canule (120) comprenant une extrémité proximale, une extrémité distale et une lumière, l'extrémité proximale de la canule (120) étant couplée à une extrémité distale du boîtier (110), l'extrémité distale de la canule (120) étant configurée pour pénétrer dans un œil d'un patient via une incision cornéenne, et la lumière de la canule (120) s'étendant de l'extrémité proximale à l'extrémité distale de la canule (120) ;
un cathéter (115) comprenant une extrémité proximale, une extrémité distale et une lumière, l'extrémité proximale du cathéter (115) étant couplée fluidiquement au réservoir (1001), et la lumière s'étendant de l'extrémité proximale du cathéter (115) à l'extrémité distale du cathéter (115) ;
un premier actionneur (105) configuré pour, lorsqu'il est actionné, faire avancer le cathéter (115) le long de la lumière de la canule (120) et amener le cathéter (115) à sortir via l'extrémité distale de la canule (120) ; et
un deuxième actionneur (128) configuré pour, lorsqu'il est actionné, amener le fluide stocké dans le réservoir (1001) à s'écouler à travers le cathéter (115) et à sortir via l'extrémité distale du cathéter (115),
dans lequel la lumière du cathéter (115) est configurée pour permettre au fluide stocké dans le réservoir (1001) de s'écouler à travers le cathéter (115) et dans l'œil du patient,
dans lequel le cathéter (115) est dimensionné de manière à s'étendre hors de l'extrémité distale de la canule (120) et le long de l'ensemble de la circonférence à 360° du canal de Schlemm du patient lorsque le premier actionneur (105) est actionné,
dans lequel le premier actionneur (105) et le deuxième actionneur (128) sont configurés pour pouvoir être actionnés par une seule main de l'utilisateur, et
dans lequel le premier actionneur (105) peut être déplacé par rapport au deuxième actionneur (128), de sorte que la distribution du fluide stocké dans le réservoir (1001) est découplée du mouvement du cathéter (115).

2. Dispositif (100) selon la revendication 1, dans lequel l'extrémité distale de la canule (120) comprend une cuillère à pointe aiguisée.

3. Dispositif (100) selon l'une quelconque des revendications 1 à 2, dans lequel l'extrémité distale de la canule (120) est pré-courbée.

4. Dispositif (100) selon l'une quelconque des revendications 1 à 3, dans lequel au moins une partie distale du cathéter (115) est préformée pour suivre approximativement un rayon de courbure du canal de Schlemm.

5. Dispositif (100) selon l'une quelconque des revendications 1 à 4, dans lequel au moins une partie distale du cathéter (115) est flexible.

6. Dispositif (100) selon l'une quelconque des revendications 1 à 5, dans lequel la canule (120) comprend un matériau rigide.

7. Dispositif (100) selon l'une quelconque des revendications 1 à 6, dans lequel l'extrémité distale de la canule (120) comprend une découpe et deux ancrages ou plus.

8. Dispositif (100) selon l'une quelconque des revendications 1 à 7, dans lequel :
le boîtier (110) comprend en outre un canal ;
le premier actionneur (105) comprend une première gâchette coulissante adaptée pour effectuer une translation axiale à l'intérieur du canal entre une position proximale et une position distale ; et
le deuxième actionneur (128) comprend une deuxième gâchette coulissante adaptée pour effectuer une translation axiale à l'intérieur du canal entre une position proximale et une position distale.

9. Dispositif (100) selon la revendication 8, dans lequel :
lorsque la première gâchette coulissante est dans la position proximale, l'extrémité distale du cathéter (115) est positionnée entre l'extrémité proximale et l'extrémité distale de la canule (120) ;
lorsque la première gâchette coulissante est dans la position distale, l'extrémité distale du cathéter (115) est avancée au-delà de l'extrémité distale de la canule (120) ;
un déplacement de la deuxième gâchette coulissante entre la position proximale et la position distale amène une quantité prédéterminée du fluide à être distribuée via l'extrémité distale du cathéter (115).

10. Dispositif (100) selon la revendication 9, dans lequel une amplitude de déplacement axial de la deuxième gâchette coulissante le long du canal correspond à une quantité du fluide distribuée depuis le cathéter (115).

11. Dispositif (100) selon la revendication 9 ou 10, dans lequel le cathéter (115) est positionné et dimensionné de telle sorte que l'extrémité distale du cathéter (115) est adaptée pour être alignée avec l'extrémité distale de la canule (120) lorsque le premier actionneur (105) est rétracté jusqu'à une position la plus proximale à l'intérieur du canal coulissant.

12. Dispositif (100) selon l'une quelconque des revendications 1 à 11, comprenant en outre un système à bille et joint à l'intérieur du connecteur (1004) configuré pour être couplé fluidiquement au dispositif viscoélastique ophtalmique (125) prérempli,
dans lequel le système à bille et joint peut permettre à un fluide de s'écouler du dispositif viscoélastique ophtalmique (125) prérempli à travers l'extrémité distale du cathéter (115) lors de l'actionnement du deuxième actionneur (128), et
dans lequel le système à bille et joint peut empêcher que le fluide ne s'écoule depuis l'extrémité distale du cathéter (115) vers le dispositif viscoélastique ophtalmique (125) prérempli.

13. Dispositif (100) selon l'une quelconque des revendications 1 à 12, dans lequel la canule (120) comprend en outre une saillie de canule le long de son extrémité distale ;
dans lequel le boîtier (110) comprend en outre une partie proximale et une partie distale ;
dans lequel la partie distale du boîtier (110) peut tourner selon un axe parallèle à une longueur de la canule (120) par rapport à la partie proximale du boîtier (110) ;
dans lequel la partie distale du boîtier (110) est reliée à la saillie de canule (120) sur la canule (120) de telle sorte qu'une rotation de la partie distale du boîtier (110) modifie une orientation de la canule (120) et donc une orientation de la partie distale de la canule (120).

14. Dispositif (100) selon l'une quelconque des revendications 1 à 13, dans lequel un déplacement du deuxième actionneur (128) par rapport au premier actionneur (105) est soumis à une résistance d'une amplitude différente de celle d'un déplacement du premier actionneur (105) par rapport au boîtier (110).
